(19) Europäisches Patentamt
European Patent Office
Office européen des brevets

(11) EP 4 067 342 B1

(12) **EUROPEAN PATENT SPECIFICATION**

(45) Date of publication and mention of the grant of the patent:
**27.12.2023 Bulletin 2023/52**

(21) Application number: **22162416.6**

(22) Date of filing: **16.03.2022**

(51) International Patent Classification (IPC):
***C07C 403/24*** *(2006.01)* ***C07C 403/12*** *(2006.01)*

(52) Cooperative Patent Classification (CPC):
**C07C 403/24; C07C 403/12;** C07C 2601/08; C07C 2601/16; Y02P 20/54

# (54) SUSTAINABLE PREPARATION OF XANTHOPHYLL ESTERS

NACHHALTIGE HERSTELLUNG VON XANTHOPHYLLESTERN

PRÉPARATION DURABLE D'ESTERS DE XANTHOPHYLLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.04.2021 LU 102731**

(43) Date of publication of application:
**05.10.2022 Bulletin 2022/40**

(73) Proprietor: **Kemijski Institut / National Institute of Chemistry**
**1000 Ljubljana (SI)**

(72) Inventors:
- **ALBREHT, Alen**
**1360 Vrhnika (SI)**
- **METLICAR, Valentina**
**3231 Grobelno (SI)**
- **KRANJC, Kristof**
**1231 Ljubljana- Crnuce (SI)**

(74) Representative: **Weickmann & Weickmann PartmbB**
**Postfach 860 820**
**81635 München (DE)**

(56) References cited:
**WO-A1-03/066583 CN-A- 103 830 113**

- **PETRY FABIANE C. ET AL: "Composition by LC-MS/MS of New Carotenoid Esters in Mango and Citrus", JOURNAL OF AGRICULTURAL AND FOOD CHEMISTRY, vol. 64, no. 43, 2 November 2016 (2016-11-02), pages 8207-8224, XP055873397, US ISSN: 0021-8561, DOI: 10.1021/acs.jafc.6b03226 ISBN: 978-4-86233-281-3**
- **SAINI RAMESH KUMAR ET AL: "Carotenoid extraction methods: A review of recent developments", FOOD CHEMISTRY, vol. 240, 1 February 2018 (2018-02-01), pages 90-103, XP055873466, NL ISSN: 0308-8146, DOI: 10.1016/j.foodchem.2017.07.099**
- **METLICAR VALENTINA ET AL: "Japanese and Bohemian Knotweeds as Sustainable Sources of Carotenoids", PLANTS, vol. 8, no. 10, 28 September 2019 (2019-09-28), page 384, XP055873467, DOI: 10.3390/plants8100384**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 067 342 B1

## Description

### Introduction

Carotenoids are natural pigments and, owing to their antioxidant,[1-3] anticancer,[1,2] antidiabetic, and anti-inflammatory activities,[2,3] they have numerous beneficial effects on human health. Carotenoids are known to reduce the risk of age-related macular degeneration,[4] protect skin, tissue, and cells from environmental toxins,[1,2] and prevent cardiovascular diseases.[1,3] In order to maintain good health these pigments must be taken up with food because carotenoids cannot be produced by the human body independently.[5,6] As secondary metabolites, carotenoids are naturally present in many plants, microorganisms and algae.[2,6] They can be divided into two subgroups: carotenes (hydrocarbons) and xanthophylls (oxygen containing derivatives). Primary biological role of xanthophylls in plants is to regulate energy dissipation during photosynthesis[7] so xanthophylls are readily found in chloroplasts of green leafy plants, including vegetables. Depending on the plant, part of the plant, and even season, xanthophylls can take two distinct forms; hydroxyl functional groups in their structure can either be free or esterified with different fatty acids.[8,9] The biological role of such esterification is still not fully understood, but it is assumed that this process enhances the chemical stability of the core carotenoid and also enables its migration from ruptured chloroplasts into chromoplasts.[10,11]

In a laboratory setting, xanthophyll esters have so far been synthesized with intent to: (i) enable their structural elucidation, (ii) alter xanthophyll's pharmacokinetic properties, (iii) increase their stability and water solubility, and (iv) prepare standard reference materials for quantitative analysis of these pigments in natural materials.[10,12] Karrer and Ishikawa were first to describe a methodology for xanthophyll ester synthesis in early 1930s using acyl chlorides in dry pyridine.[13,14] Later, this procedure underwent many alterations where different reaction solvents, catalysts, and fatty acids or derivatives thereof were used. Many aliphatic, amphipathic, ionic (charged and neutral) and different bifunctional xanthophyll esters were also prepared. For a comprehensive review on esterification strategies and functionalities of xanthophyll esters, the reader is directed to a recent monograph by Nagy et al.[12] Enzyme catalysed esterification with immobilised lipases could be considered as the current state of the art in the field, but application of hazardous organic solvents (tetrahydrofuran, toluene, hexane), long reaction times, increased temperatures, and low reaction yields render this seemingly green approach impractical for a more general utilization.[15-17] And not only those involving enzymatic catalysis, but in general, known synthetic pathways to xanthophyll esters are not environmentally friendly and economically viable on an industrial scale;[12] many are energetically non-efficient (heating up to 90 °C),[18,19] all employ either halogenated, aromatic, or other solvents and chemicals which are hazardous to the environment and to the human health and cannot be easily recycled, they generate large amounts of waste, do not use renewable feedstocks, and lastly, many degradation, isomerization, and other side reactions are often taking place, resulting in poor yield of the esterification reaction.[12-14,20-23] The latter is especially true when acyl chlorides are used as the esterifying agents.[12,20] Carotenoids, including hydroxylated xanthophylls, are well-known for their instability when exposed to light, oxidants, acids, elevated temperatures, or trace levels of metals.[5,24] This inherent property sets xanthophylls apart from most alcohols and introduces complexity into a simple reaction such as esterification. Moreover, reactivity of different xanthophylls was shown to vary under otherwise identical experimental conditions, which makes a development of a universal synthetic approach quite challenging.[12,20,25]A few synthetic routes were patented,[18,19,26-28] but these are hampered primarily by the use of toxic halogenated solvents, high energy consumption,[18,19] and low reaction yields (30-80%).[26]. WO03/066583 discloses the esterification of xanthophyll with an acid derivative, being acid chloride or acid anhydride, in presence of a base being triethylamine, pyridine or DMAP, and in solvents selected from DCM; acetone, dioxane, ethers, aromatics. CN103830113 discloses reacting astaxanthin with succinic anhydride in either the absence of solvent at 50°C or in acetone at 60°C.

Last, but not least, strategies for purification of the reaction product(s) are scarce and in many cases suffer from the same shortcomings discussed above. Additionally, purification typically requires multiple steps with negative impacts on product yields.[29] In extreme cases, such product processing is omitted altogether, posing a serious health risk, especially when the synthesized xanthophyll ester is to be used in the food, feed, or food supplement industry.

The demand for carotenoids as health promoting nutrients is high and increasing as demonstrated by the technological innovations in the field and by a growing 1.5 billion USD carotenoid market size (2019).[30] However, rapid decomposition of these pigments and their short shelf-life remain a challenge to be solved, despite decades of intense chemical exploration. Xanthophyll esters were shown to have superior stability and bioavailability compared to their free forms so green approaches for the preparation of such esters are of great industrial interest since these would support economic and environmental sustainability.[31-33]

Thus, the problem underlying the present invention was to provide a method for the sustainable preparation of xanthophyll esters. The main purpose was to overcome persisting drawbacks and streamline the first esterification of different xanthophylls while adhering to the basic principles of green chemistry.[34] In particular, the term "green solvent" is known to the person skilled in the art of extraction methodology and refers to solvents with the lowest toxicity such as acetone, ethanol, methanol, 2-propanol, ethyl-acetate, isopropanyl-acetate, methyl ethyl ketone, 1-butanol and tert-butanol. Green solvents are thus environmentally friendly solvents or even biosolvents which may in some embodiments be derived from the processing of agricultural crops. A systematic approach was used to optimise all relevant stages and not only the mere synthesis. These are: (i) the selection of starting reagents/materials, (ii) the esterification reaction, and (iii) product purification. The use of reusable, renewable, and GRAS (Generally Recognised As Safe) materials was preferred. A balance between the efficiency of xanthophyll ester production and greenness of the platform was established.

The present invention provides a method for preparing xanthophyll ester, comprising

(i) providing at least one xanthophyll comprising at least one hydroxyl (OH) functional group,
(ii) esterification of at least one xanthophyll with acid anhydride in the presence of 4-dimethylaminopyridin (DMAP), wherein the esterification is carried out in the absence of solvent or in the presence of a solvent selected from β-pinene, and ethyl acetate (EtOAc), wherein the esterification in step (ii) is carried out at a reaction temperature in the range of 18-25°C, and
(iii) isolating xanthophyll ester from the reaction mixture.

Previous studies show that esterification with acyl chlorides results in the formation of various unwanted side products.[12,20,35] Poor reaction selectivity and yields in those cases are ascribed mainly to *Z-E*-isomerisation and degradation of labile xanthophylls. Both events are likely to be caused by hydrochloric acid, which is released during the reaction. Thus, the method of the present invention uses acid anhydrides as esterifying agents. The anhydrides can be derived from aromatic, (non)functionalized, (non)branched, (un)saturated short- and long-chain aliphatic carboxylic acids. Exemplary acid anhydrides for use in the present invention are shown in Table 1.

In principle, any type of xanthophyll comprising at least one OH-functional group can be used in the method of the invention. In the present application, the term "xanthophyll" is used synonymously for hydroxyl-group-containing xanthophyll. Examples of xanthophylls are monohydroxyxanthophyll (β-cryptoxanthin) and dihydroxyxanthophylls (lutein, zeaxanthin, capsanthin, and violaxanthin) (Table 1).

**Table 1.** Xanthophylls and acid anhydrides

| Compound | Molecular formula | Chemical structure |
|---|---|---|
| **Xanthophylls** | | |
| Capsanthin | $C_{40}H_{56}O_3$ | 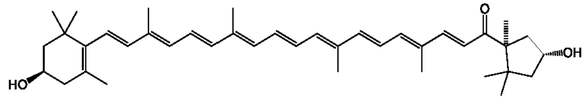 |
| P-Cryptoxanthin | $C_{40}H_{56}O$ | 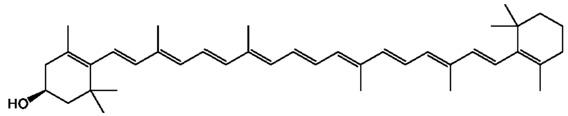 |
| Lutein | $C_{40}H_{56}O_2$ | 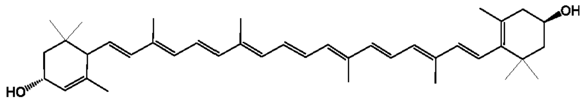 |
| Zeaxanthin | $C_{40}H_{56}O_2$ | 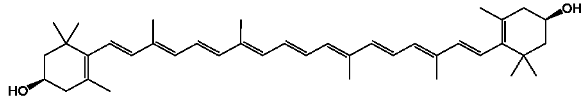 |
| Violaxanthin | $C_{40}H_{56}O_4$ | 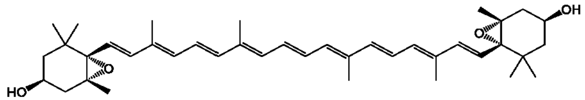 |
| **Acid anhydrides ($R_2O$)** | | |
| **Acid anhydride** | **Molecular formula** | **R =** |
| Acetic | $(CH_3CO)_2O$ | 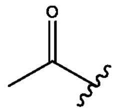 |
| Propionic | $(CH_3CH_2CO)_2O$ | 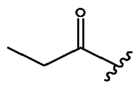 |

(continued)

| Acid anhydrides ($R_2O$) | | |
|---|---|---|
| **Acid anhydride** | **Molecular formula** | **R =** |
| 2,2-Dimethylpropionic | $(CH_3)_3CCO)_2O$ | 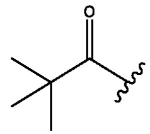 |
| Valeric | $(CH_3(CH_2)_3CO)_2O$ | 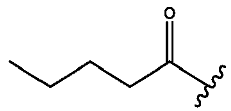 |
| Pent-4-enoic | $(H_2C{=}CHCH_2CH_2CO\ )_2O$ | 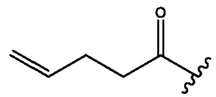 |
| Benzoic | $(C_6H_5CO)_2O$ | 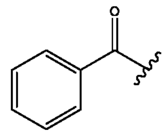 |
| Decanoic | $(CH_3(CH_2)_8CO)_2O$ | 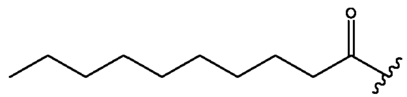 |
| Palmitic | $(CH_3(CH_2)_{14}CO)_2O$ | 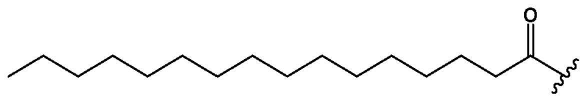 |
| Oleic | $(CH_3(CH_2)_7CH{=}CH\ (CH_2)_7CO)_2O$ | 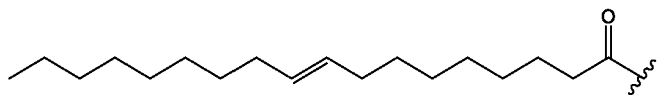 |
| Pentafluoropropionic | $(CF_3CF_2CO)_2O$ | 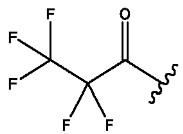 |

(continued)

| Acid anhydrides ($R_2O$) | | |
|---|---|---|
| **Acid anhydride** | **Molecular formula** | **R =** |
| Phthalic | $C_6H_4(CO)_2O$ | 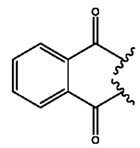 |

Xanthophyll can be used in pure form or as an extract obtained from plant material containing xanthophyll. Thus, xanthophyll does not necessarily have to be isolated from the extract, but it is possible to use the (raw) extract directly. Suitable plant materials are for example peels of banana, mango, and/or avocado and green leaves of Japanese knotweed, Bohemian knotweed or giant knotweed. Renewable feedstock as a source of free xanthophylls enables industrial applications in support of a sustainable and circular economy. What is more, the exhaustion of invasive alien plant species such as Japanese knotweed is actually even encouraged in order to ensure biodiversity within our ecosystem. Utilization of fruit peels supports Zero Waste and a reduction in $CO_2$ emissions.

According to a preferred embodiment, the extract is provided by extracting banana, mango, and/or avocado peels and/or green leaves of Japanese knotweed, Bohemian knotweed or giant knotweed using β-pinene, acetone and/or EtOAc or supercritical $CO_2$ as extracting agent, preferably in nitrogen atmosphere and/or under reduced light conditions.

The esterification in step (ii) is carried out with DMAP as a catalyst. It was found, that DMAP is superior to other catalysts commonly used in esterification reactions. DMAP can be used in free form or optionally in polymer-supported form (pDMAP).

The stoichiometry of reagents in the esterification reaction preferably includes a molar ratio corresponding to at least 1.5 eq. of acid anhydride for each of the xanthophyll OH groups. Higher amounts of acid anhydride are preferred to achieve maximum conversion of the xanthophyll and avoid side products. For example, a molar ratio corresponding to at least 2.5, 3.0, 5.0, or 7.5 eq. of acid anhydride per xanthophyll OH group can be used. On the other hand, the excess of anhydride should not be too large for ecological reasons, in particular not more than 10 eq. of acid anhydride per xanthophyll OH group.

The molar ratio of acid anhydride : DMAP or pDMAP is preferably in a range of 1 : 1.5 to 1 : 3, in particular about 1 : 2.

The reaction temperature in step (ii) has an appreciable effect on the reaction rate, equilibrium, and chemical stability of free (reactant) and esterified xanthophylls (product).[35,41,42] At elevated temperatures (60 °C or 80 °C), the rates of xanthophyll diacetate formation are high. However, after some time, the yields significantly drop due to the sensitivity of the pigment towards prolonged exposures to increased temperatures. Thus, in the method of the present invention, a temperature around ambient temperature in the range of 18-25°C was found to be particularly suitable. Preferably, the temperature is in the range of 20-25 °C. At these temperatures, the product yields remain stable and quantitative even after 24 h.

In order to minimize xanthophyll degradation, the esterification reaction in step (ii) is preferably carried out in nitrogen atmosphere and/or under reduced light conditions.

The esterification reaction can be carried out solvent-free or in the presence of a solvent. The method of the present invention uses a green and/or a GRAS reaction solvent selected from β-pinene, and EtOAc. These solvents were found to surprisingly provide highest yields of the desired xanthophyll diesters. β-Pinene is considered especially suitable as a reaction solvent since this terpene is obtained entirely from renewable feedstock, it poses fewer hazard risks, is inexpensive, and has a much smaller environmental impact compared to common petrol-derived hydrocarbons such as n-hexane, for instance.[48] According to the European Chemicals Agency β-pinene is readily biodegradable in water. Together with its structural isomer α-pinene, β-pinene is the major component of turpentine oil obtained from many conifers, with pine tree being the most representative.[49] Moreover, β-pinene is permitted by FDA for direct addition to food for human consumption (Code Of Federal Regulations 21CFR172.515), it is regarded as a safe food additive by JECFA (Joint FAO/WHO Expert Committee on Food Additives), and even has particular positive effects on human health.[49]

In step (iii), xanthophyll ester is isolated from the reaction mixture. Preferably, isolating xanthophyll ester comprises liquid-liquid extraction (LLE) and/or solid phase extraction (SPE). LLE approach is greener, faster, simpler, and less expensive. On the other hand, SPE is truly universal and offers products with high degrees of purity, but slightly larger product loses are encountered during the purification and more waste is created (SPE cartridges, organic solvents). According to a preferred embodiment of the invention, isolation of xanthophyll ester comprises liquid-liquid extraction using water or an alkaline aqueous solution, such as aqueous NaHCOs. A slightly alkaline aqueous solution is best at simultaneously removing excess DMAP, acid anhydrides, and their corresponding acids, but water can also readily be used.

The beneficial results like high yields for the esterification described herein, i.e. in particular esterification with acid anhydride in the presence of DMAP and in the absence of solvent or in the presence of green solvent (i.e. β-pinene of ethyl acetate) are absolutely surprising and not rendered obvious by any prior art.

Using the method according to the present invention, it was possible to synthesize the following compounds for the first time: violaxanthin dioleate, violaxanthin dipropanoate, violaxanthin di(2,2-dimethylpropanoate), violaxanthin di(pentafluoropropanoate), violaxanthin diphthalate, violaxanthin di(pent-4-enoate), capsanthin di(2,2-dimethylpropanoate), capsanthin di(pentafluoropropanoate), capsanthin diphthalate, capsanthin di(pent-4-enoate), lutein diphthalate, lutein di(pentafluoropropanoate), lutein di(pent-4-enoate), zeaxanthin diphthalate, zeaxanthin di(pentafluoropropanoate), zeaxanthin di(pent-4-enoate), β-cryptoxanthin 2,2-dimethylpropanoate, β-cryptoxanthin pentafluoropropanoate, β-cryptoxanthin phthalate, β-cryptoxanthin pent-4-enoate, β-cryptoxanthin benzoate, β-cryptoxanthin valerate.

The invention will be further illustrated with reference to the following figures and examples.

## Figures

**Fig. 1** Formation of lutein diacetate when using different catalysts; DMAP (diamond), polymer supported DMAP (square), Ambersep 900 hydroxide (triangle), TEA (cross), and pyridine (asterisk). Molar ratio between lutein : acetic anhydride : DMAP was 1 : 15 : 30. Reaction with lutein (0.3 mM) was carried out in the dark under nitrogen atmosphere at 40 °C and EtOAc was used as the reaction solvent.

**Fig. 2** Depletion of lutein and formation of lutein esters over time in the presence of DMAP (A) and polymer supported DMAP (B); free lutein (diamond), lutein monoacetate (square), lutein diacetate (triangle), and Z-lutein diacetate (cross). Molar ratio between lutein : acetic anhydride : catalyst was 1 : 15 : 30. Reaction with lutein (0.3 mM) was carried out in the dark under nitrogen atmosphere at 40 °C and EtOAc was used as the reaction solvent.

**Fig. 3** Yield of lutein diacetate when recycled pDMAP was used; 1st use of base (diamond), 1st reuse (square), 2nd reuse (triangle), 3rd reuse (cross) and 4th reuse (plus). Molar ratio between lutein : acetic anhydride : catalyst was 1 : 15 : 30. Reaction with lutein (2.3 mM) was carried out in the dark under nitrogen atmosphere at 40 °C and EtOAc was used as the reaction solvent. After each cycle the pDMAP resin (10 mg) was rigorously washed in 8 steps: 1. and 2.) iso-propanol : MQ water (1:1, 4 mL), 3.) iso-propanol : NaOH (2M) (1:5; 2 mL) mixture, and 4.) iso-propanol : MQ water (1:5, 2 mL) and 5. - 8.) MQ water (2 mL) (repeated 2 times).

**Fig. 4** Depletion of lutein and formation of lutein esters over time at lutein : acetic anhydride : DMAP molar stoichiometric ratios of 1 : 15 : 30 (A), 1 :10 : 20 (B), and 1 : 5 : 10 (C); lutein (diamond), lutein monoacetate (square), lutein diacetate (triangle). Reaction with lutein (0.3 mM) was carried out in the dark under nitrogen atmosphere at 40 °C and EtOAc was used as the reaction solvent.

**Fig. 5** Depletion of lutein and formation of lutein esters at high lutein concentration (2.3 mM) and lutein : acetic anhydride : DMAP molar stoichiometric ratios of 1 : 3 : 6 (A) and 1 : 6 : 12 (B); lutein (diamond), lutein monoacetate (square), lutein diacetate (triangle). Reaction was carried out in the dark under nitrogen atmosphere at 40 °C and EtOAc was used as the reaction solvent.

**Fig. 6** Influence of temperature on the reaction rate and stability of lutein diacetate; ambient temperature (diamond), 40 °C (square), 60 °C (triangle) and 80 °C (cross). Reaction with lutein (0.3 mM) was carried out in the dark under nitrogen atmosphere. EtOAc was used as a reaction solvent and lutein : acetic anhydride : DMAP molar stoichiometric ratio was 1 : 15 : 30.

**Fig. 7** Solvent effect on the formation of lutein diacetate (**A**), lutein monoacetate (**B**), and depletion of lutein (**C**); α-terpinene (diamond), γ-terpinene (square), D-limonene (triangle), β-pinene (cross), α-pinene (plus), EtOAc (asterisk), and acetone (circle). Molar stoichiometric ratio of lutein : acetic anhydride : DMAP was 1 : 15 : 30. Reaction with lutein (0.3 mM) was carried out in the dark under nitrogen atmosphere at ambient temperature.

**Fig. 8** Depletion of lutein and lutein esters formation in solvent-free reaction with solid (**A**) or liquid (**B**) acid anhydrides at lutein : acid anhydride : DMAP molar stoichiometric ratios of 1 : 15 : 30. Reaction with lutein (1 mg) was performed in a mortar (20 min) at room temperature.

**Fig. 9** HPTLC chromatogram of violaxanthin diacetate (1), dipropanoate (2), di(2,2-dimethylpropanoate) (3), divalerate (4), di(pent-4-enoate) (5), dibenzoate (6), didecanoate (7), dipalmitate (8), dioleate (9), di(pentafluoropropanoate) (10), diphthalate (11), and violaxanthin standard solution (12). C18 HPTLC silica gel plate was predeveloped with MeOH : dichloromethane 1 : 1 (*v*/*v*). Crude reaction mixtures from violaxanthin esterifications (75 μL) and violaxanthin standard mix solution (5 μL, *c* = 50 μg/mL) were applied on the plate by means of Linomat 5 (Muttenz, Switzerland). Then, the plate was developed using acetone : methanol (1 : 1, *v*/*v*) + 0.1% TBHQ in a saturated twin trough chamber. The images were acquired using white light in transmission mode before (top) and immediately after exposure to HCl vapours (bottom).

**Fig. 10** HPLC chromatograms of extracts from unripe orange peels (A), ripe orange peels (B), unripe lemon peels (C), ripe lemon peels (D), unripe banana peels (E), ripe banana peels (F), unripe mandarin peels (G), ripe mandarin peels (H), unripe mango peels (I), and ripe avocado peels (J): violaxanthin (1), antheraxanthin (2), lutein (3), β-cryptoxanthin (4), (9*Z*)-β-carotene (5), α-carotene (6), β-carotene (7). Xanthophyll esters (mainly of violaxanthin and lutein) are highlighted with dotted braces. Absorbance scales were normalized in each case to the largest chromatographic peak in order to fully appreciate the xanthophyll profiles of peel extracts. Compounds were separated on a ProntoSIL C30 column (250 mm × 4.6 mm i.d., 5 μm) from Bischoff (Leonberg, Germany). Gradient elution of compounds was performed from 90% to 100% acetone. The flow rate was 0.8 mL/min, data was acquired at 450 nm, and column temperature was maintained at 35 °C. Injection volume of 5-fold diluted crude extracts was 10 μL.

**Fig. 11** HPTLC chromatograms of β-pinene extract (A), acetone extract (B), EtOAc extract (C), and sc-$CO_2$ extract (dissolved in β-pinene) (D) of Japanese knotweed green leaves: β-carotene (1), pheophytin (b/b' or a/a') (2), chlorophyll b/b' (3) and chlorophyll a/a' (4), lutein + zeaxanthin (5), neoxanthin (6), and violaxanthin (7). C18 HPTLC silica gel plate was predeveloped with MeOH : dichloromethane 1 : 1 (*v*/*v*). Japanese knotweed leaf extracts (15 μL) and sc-$CO_2$ extract in β-pinene (5 μL) were applied on the plate by means of Linomat 5. Then, the plate was developed using acetone : methanol (1 : 1 *v*/*v*) + 0.1% TBHQ in a saturated twin trough chamber. The images were acquired using white light in transmission mode and under 254 nm and 366 nm.

**Fig. 12** HPTLC densitograms of EtOAc extract (A), acetone extract (B), β-pinene extract (C), and sc-$CO_2$ extract (D) of green leaves of Japanese knotweed scanned at 254 nm, 280 nm and 366 nm in absorption/reflectance mode. C18 HPTLC silica gel plate was predeveloped with MeOH : dichloromethane 1:1 (*v*/*v*). Japanese knotweed leaf extracts (15 μL) and sc-$CO_2$ extract in β-pinene (5 μL) were applied on the plate by means of Linomat 5. Then, the plate was developed using acetone : methanol (1:1 *v*/*v*) + 0.1% TBHQ in a saturated twin trough chamber.

**Fig. 13** Effect of interferences from the Japanese knotweed extract's matrices on the formation of lutein diacetate; β-pinene extract (diamond), EtOAc extract (square),and sc-$CO_2$ extract (triangle). Solid residue of EtOAc extract (after solvent removal) and sc-$CO_2$ extract were re-dissolved in β-pinene and all reactions were carried out in the dark under nitrogen atmosphere at ambient temperature at lutein : acetic anhydride : DMAP molar stoichiometric ratios of 1 : 70 : 130 (A), 1 : 90 : 150 (B), and 1 : 120 : 200 (C).

## Examples

### 1. Materials and methods

### 1.1. Chemicals and standards

All solvents and reagents were at least of analytical grade. Methanol (MeOH; LC-MS grade), acetonitrile (ACN), 2-*tert*-butylhydroquinone (TBHQ, 97 %) and acetone were supplied by Honeywell (Seelze, Germany). Ethanol (EtOH) was from Carlo Erba (Val-de-Reuil, France). Acetone (LC-MS grade), ethyl acetate (EtOAc), isopropanol, methyl *tert*-butyl ether (MTBE), orthophosphoric acid (85%), dry pyridine (>99.8%), hydrochloric acid (37%), sodium chloride (NaCl), sodium hydrogen carbonate (NaHCOs), thymol (99%), dichloromethane (DCM), pentanol (98.5%) and zinc chloride (98%) were from Merck (Darmstadt, Germany). Ion exchange resin Ambersep 900 hydroxide form, octanol (>99.5%), α-terpinene (90%), γ-terpinene (97%) and (-)-α-pinene (≥99%), were from Fluka Analytical (Steinheim, Germany). Triethylamine (TEA; 99.5%), 4-dimethylaminopyridine (DMAP), polymer supported 4-dimethylaminopyridine (3 mmol/g DMAP loading), (+)-limonene (97%), (-)-β-pinene (99%), menthol (99%), coumarin (≥99%), lidocaine (99%), choline chloride (99%), citric acid (99%), decanoic acid (≥98%), malic acid (≥98%) and all anhydrides: acetic (99%), propionic (≥99%), valeric (97%), pent-4-enoic (98%), 2,2-dimethylpropionic (99%), pentafluoropropionic (≥99%), phthalic (≥99%), benzoic (≥95%), decanoic (96%), oleic (95%) and palmitic (97%), were purchased from Sigma-Aldrich (St. Louis, MO, USA). Retinol (99%) was from Serva (Heidelberg, Germany) and 1-decanol (98%) was from Kemika (Zagreb, Croatia). All-E-capsanthin (≥95%), all-E-zeaxanthin (≥98%), and all-E-β-cryptoxanthin (≥97%) were acquired from Extrasynthèse (Genay, France), while all-E-lutein (95%) and all-E-violaxanthin (≥90%) were from Sigma-Aldrich. Xanthophylls and acid anhydrides used in the present invention are listed in Table 1. If not stated otherwise, xanthophylls were assumed to maintain an (all-E)-configuration throughout. Ultrapure Milli-Q water (18 MΩ $cm^{-1}$) was supplied by a Milli-Q water purification system (Millipore, Bedford, MA, USA). Solvents, which were used to prepare plant material extracts, standard solutions, and reagent solutions were purged with nitrogen before use.

### 1.2. Extraction of carotenoids from plant materials

Peels of ripe and unripe fruits were obtained from orange (*Citrus × sinensis*), mandarin (*Citrus reticulata*), banana (Musa (genus)), lemon (*Citrus × limon*), mango (*Mangifera indica*), and avocado (*Persea americana*) purchased at a local market, whereas green leaves of Japanese knotweed (*Fallopia japonica* Houtt.) were harvested in September 2019 in the area of Ljubljana. The plant materials were frozen in liquid nitrogen, lyophilized, and pulverized. The resulting material was then accurately weighed (300 mg) and extracted with either EtOAc (10 mL) or β-pinene (10 mL) for 15 min in the Carousel 12 Plus apparatus (Radleys, Safron Walden, UK). The extraction was carried out at ambient temperature under a nitrogen atmosphere and under reduced light conditions. Afterwards, the extracts were centrifuged at 1800 g for 5 min and the supernatant was additionally filtered through a 0.2 μm polyvinylidene fluoride (PVDF) membrane filter (LLG labware, Meckenheim, Germany). Prepared extracts were stored in amber glass vials (National Scientific Company, USA) at -80 °C before further use.

Extraction of xanthophylls from the leaves of Japanese knotweed was also performed by means of supercritical $CO_2$ (sc-$CO_2$). The extraction cell was loaded with dried pulverized plant material (5 kg). The extraction was carried out at 150 bar, 65 °C and at flow rate of 120 g $CO_2$/min. The sc-$CO_2$ extract (65.56 g) was collected from the separator, which was maintained at 80 bar and 45 °C during operation. A small aliquot of the obtained sc-$CO_2$ extract was accurately weighed in an amber glass vial (50 mg), dissolved in β-pinene (10 mL) and stored at -80 °C before further use.

### 1.3. Preparation of standard and reagent solutions

Capsanthin, β-cryptoxanthin, lutein, zeaxanthin, and violaxanthin stock solutions were prepared by dissolving individual xanthophylls in acetone. Exact concentration of each prepared stock solution (400-450 μg/mL) was determined spectrophotometrically through their known absorption coefficients by using the Beer-Lambert law. Solutions were stored in amber glass vials at -80 °C before further use. Xanthophyll standard solutions used for high-performance liquid chromatographic (HPLC) and high-performance thin-layer chromatographic (HPTLC) analyses were prepared by further diluting the standard stock solutions with acetone (final concentration 40-45 μg/mL). DMAP stock solution (60 mmol/L) and anhydride stock solution (30 mmol/L) were prepared individually in β-pinene and stored at 6 °C before use. For reactions carried out on plant extracts (*vide infra*), 10-fold higher concentrations of DMAP and acid anhydride were used (600 and 300 mmol/L, respectively).

### 1.4. Synthesis of xanthophyll esters

In a typical esterification reaction, a suitable volume of xanthophyll stock solution (corresponding to 0.2 μmol) was transferred to the reaction vessel. Solvent was removed under a stream of nitrogen and the solid residue was re-dissolved in β-pinene (200 μL), however, EtOAc could also readily be used. Then, both DMAP solution (6 μmol; 250 μL) and the corresponding anhydride solution (3 μmol; 250 μL) were added (450 μmol of DMAP and 450 μmol of anhydride in the case of 2,2-dimethylpropionic and pentafluoropropionic anhydrides). The mixture (700 μL in total) was stirred at ambient temperature (22 °C) in an inert nitrogen atmosphere and in the absence of light for 24 h. Afterwards, the esterified xanthophyll product was purified either by means of liquid-liquid extraction (LLE) or by solid phase extraction (SPE).

When extracts from natural materials were used as xanthophyll sources, stock solutions of DMAP (600 mmol/L, 50 μL) and acid anhydride (300 mmol/L, 50 μL) were added directly to the extract (600 μL) and then the reaction was carried out as described above. In the case of solvent-free esterification, lutein was weighed directly into the reaction vessel (2 μmol), then DMAP (60 μmol) and acid anhydride (30 μmol) either in liquid or solid form were added and the mixture was mechanically mixed for 20 min at ambient temperature and pressure. Purification of lutein ester products was carried out as described below.

### 1.5. Purification of xanthophyll esters

#### 1.5.1. Liquid-liquid extraction

The crude reaction mixture (300 μL) was pipetted into a polypropylene tube (2 mL; Sarstedt AG & CO, Nümbrecht, Germany) and the same volume of 10% $NaHCO_{3(aq)}$ was added. The mixture was vortexed for 2 min and then centrifuged at 1000 g for 2 min. The aqueous phase was removed and the procedure was repeated two more times. Crude reaction mixture, the organic phase after the final wash, and aqueous phases after each washing step were analyzed by HPLC for products, side-products, and reactants to determine the xanthophyll ester yield and purity.

### 1.5.2. Solid phase extraction

Xanthophyll esters were purified in parallel also on C18 SPE cartridges (3CC/200mg) from Varian (Harbor City, USA). The flow rate of solvents applied to the SPE cartridges was 1 mL/min (0.5 mL/min for sample loading) and was regulated by a vacuum pump. The following SPE procedure was used: (i) cartridges were conditioned first with acetone (3 mL) and then with an equal volume of isopropanol : EtOAc : water (1 : 1 : *1; v/v/v),* (ii) crude reaction mixture (100 μL) was diluted 10-fold with 85% EtOH and loaded onto the cartridge, (iii) the cartridge was washed with 80% EtOH (0.1% $NH_3$ in 95% EtOH for xanthophyll palmitates and oleates) (3 mL), and finally (iv) the synthesized xanthophyll ester was eluted with acetone (2 mL). The volatile components from the eluate were removed under a stream of nitrogen and dry residue was subsequently re-dissolved in ACN (0.5 mL) for HPLC analysis to determine the xanthophyll ester yield and purity.

### Results and discussion

The present invention describes the first multifaceted yet tuneable green synthetic platform for the preparation of xanthophyll esters. One monohydroxyxanthophyll (β-cryptoxanthin) and four dihydroxyxanthophylls (lutein, zeaxanthin, capsanthin, and violaxanthin) were used to create a library of esterified xanthophylls by various carboxylic acids: aromatic, (non)functionalized, (non)branched, (un)saturated short- and long-chain aliphatic carboxylic acids (Table 1). Previous studies show that esterification with acyl chlorides results in the formation of various unwanted side products.[12,20,35] Poor reaction selectivity and yields in those cases are ascribed mainly to *Z*/*E*-isomerisation and degradation of labile xanthophylls. Both events are likely to be caused by hydrochloric acid which is released during the reaction. Thus, here we use acid anhydrides as esterifying agents on account of lower acidity of their corresponding carboxylic acids formed as side products (in comparison with hydrochloric acid). The selection of 11 acid anhydrides was guided by diversity to demonstrate a wide applicability of the developed synthetic platform. However, a combination of lutein and acetic anhydride was initially used as a model system for a systematic optimisation of the reaction in terms of greenness and performance.

### 2.1. Optimization of xanthophyll esterification

The following examples performed during optimisation of the esterification conditions and using different solvents than β-pinene and ethyl acetate , different bases than DMAP or pDMAP , or temperatures higher than 25°C are not according to the invention and are present for illustration purposes only.

Type and amount of a catalyst, stoichiometry of reagents, temperature, and reaction solvent are four main parameters subjected to optimization. Taken as a starting-point, lutein was converted into lutein diacetate (desired product) with a 7.5x excess of acetic anhydride in the presence of DMAP at 40 °C in EtOAc. The reaction was carried out in nitrogen atmosphere and under reduced light conditions in order to minimize xanthophyll degradation.

### 2.1.1. Catalyst

Bases such as pyridine,[13,14,22] TEA,[36,37] and DMAP[25,38,39] are well-known, easy-to-handle catalysts, capable of neutralizing acids formed during various synthetic procedures including the esterification reactions of xanthophylls. In the present invention, we additionally explored a strong anion exchanger Ambersep 900 hydroxide (a quaternary ammonium functionality) and a polymer-bound DMAP because both materials can be reused. By using strong anion exchange resin the reaction yielded no product thus demonstrating that mere neutralization of the carboxylic acid formed during the reaction was not sufficient to shift the equilibrium towards a successful esterification (Fig. 1). By employing pyridine, 10% of lutein diacetate was obtained after 24 h, while merely traces of lutein monoesters were detected with TEA. Finally, DMAP and polymer supported DMAP (pDMAP) proved most promising. An excellent yield of 95% for lutein diacetate was obtained after 4 h when using DMAP (lutein : acetic anhydride : DMAP = 1 : 15 : 30) and only 4% of lutein monoacetates formed in the process. On the other hand, the same molar amount of pDMAP yielded only 55% of the expected lutein diacetate with 15% of lutein monoacetates as side products, even after 24 h. Furthermore, 11% of the synthesized lutein diacetate isomerized into one of its Z-isomers while this unwanted side product amounted to less than 2% when non-bound DMAP was used (Fig. 2). Reasons for such performance differences between DMAP and pDMAP remain unclear, but steric hindrance induced by the support could at least partially explain the compromised yield. A significant advantage of pDMAP is that it can be recovered and reused although the performance of the catalyst appreciably deteriorated within only 5 cycles, dropping the yield from 75% to 33% (Fig. 3). Regeneration of pDMAP involved washing the resin in 8 steps with a sodium hydroxide solution in combination with isopropanol/water which introduced additional costs and generated waste (16 mL of solvent waste per 10 mg of pDMAP). Thus, the advantages of such a seemingly green solution should be carefully weighed on a case-by-case basis. We decided to use non-bound

DMAP as a way forward as an overall greener option.

### 2.1.2. Stoichiometry of reagents

It is not uncommon to use a large excess of an esterifying agent (up to 25 fold) to drive conversions of xanthophylls to their corresponding esters to (near) completion.[17,111,26,27,40] On average, 10-20 equivalents (eq) of the reagent are used. However, by doing so, large amounts of waste are created. In the present invention, a total conversion of lutein (0.3 mM in EtOAc) into its diacetate was observed after 6 h when lutein : acetic anhydride : DMAP were reacted in a molar ratio of 1 : 15 : 30 (7.5 eq of anhydride for each of the lutein OH groups); large excess of DMAP was necessary to avoid isomerization and degradation of pigments. With lower ratios (i.e., 1 : 10 : 20 and 1 : 5 : 10) lutein monoacetates were obtained as side products (amounting to 20-50% of all products) and lutein was not entirely consumed (Fig. 4). However, if we increased the concentration of lutein to 2.3 mM the reaction yields for lutein diacetate were as high as 84% and 92% even at low molar ratios such as 1 : 3 : 6 and 1 : 6 : 12, respectively (Fig. 5). This is the first report of a successful synthesis of any xanthophyll diester where as little as 1.5 eq of an esterifying agent was used. Nonetheless, the limited amounts of xanthophyll compounds at our disposal necessitated all further reactions to be carried out at a sub-optimal (0.3 mM) concentration level (with 7.5 eq of acid anhydrides) in order to successfully prepare and compare all 55 anticipated products (from 5 xanthophylls and 11 acid anhydrides) under the same experimental conditions.

### 2.1.3. Temperature

The temperature has an appreciable effect on the reaction rate, equilibrium, and chemical stability of free (reactant) and esterified xanthophylls (product).[35,41,42] At elevated temperatures (60 °C or 80 °C), the rates of lutein diacetate formation were high, but maximum reaction yields of only 52% (at 80 °C after 1 h) and 84% (at 60 °C after 4 h) were obtained (Fig. 6). After 24 h, the yields dropped below 10%, which clearly indicated the sensitivity of the pigment towards prolonged exposures to increased temperatures. At 40 °C and at ambient temperature the reaction rates were lower, but yields remained stable and quantitative even after 24 h. In other studies, reactions were most often carried out at ambient temperatures (20-25 °C) or at 40 °C.[16,20,21,27,36,37,43] In some instances, higher temperatures were also used (50-90 °C), but no product degradation was reported.[17-19,40,42] We show that ambient temperature is the most energy efficient option which ensures good yields with concomitant product stability.

### 2.1.4. Reaction solvent

Hazardous and harmful reaction solvents such as n-hexane, benzene, toluene, chloroform, dichloromethane, and tetrahydrofuran have typically been used to prepare xanthophyll esters because xanthophylls are highly soluble in these solvents and practically insoluble in water and other polar solvents. Since the reaction solvent represents the majority of the mass (and waste) in a batch chemical synthesis, our aim was to introduce a green and/or a GRAS reaction solvent. Two green organic solvents (acetone and EtOAc)[44-46] were considered as well as five bio-derived monoterpenes (α-pinene, β-pinene, α-terpinene, γ-terpinene, and D-limonene) which have the potential to substitute halogenated hydrocarbons.[47] Out of the 7 candidates, only β-pinene, acetone, and EtOAc enabled lutein diacetate yields above 85% (Fig. 7), but in the case of acetone, 5% of side products were additionally obtained. β-Pinene was considered especially suitable as a reaction solvent since this terpene is obtained entirely from renewable feedstock, it poses fewer hazard risks, is inexpensive, and has a much smaller environmental impact compared to common petrol-derived hydrocarbons such as *n*-hexane, for instance.[48] According to the European Chemicals Agency β-pinene is readily biodegradable in water. Together with its structural isomer α-pinene, β-pinene is the major component of turpentine oil obtained from many conifers, with pine tree being the most representative.[49] Moreover, β-pinene is permitted by FDA for direct addition to food for human consumption (Code Of Federal Regulations 21CFR172.515), it is regarded as a safe food additive by JECFA (Joint FAO/WHO Expert Committee on Food Additives), and even has particular positive effects on human health.[49]

Other green solvents such as hydrophobic natural deep eutectic solvents (NADESs)[50-52] were also evaluated as reaction media (menthol-thymol 1 : 1, menthol-decanoic acid 1 : 1, thymol-decanoic acid 1 : 1, thymol-coumarin 1 : 1, lidocaine-decanoic acid 1 : 2, and choline chloride-malic acid 1 : 1). Although all NADESs were successful in solubilizing the hydrophobic xanthophylls, they did not meet the chemical inertness criteria. Being in molar excess of several orders of magnitude relative to the xanthophyll, they competitively reacted with the acid anhydride, thus, depleting it. Another DES - choline chloride-$ZnCl_2$ 1 : 2, which has previously been used in similar esterification reactions involving anhydrides,[53] also proved unsuccessful as it did not dissolve any of the studied hydrophobic xanthophylls. Finally, it should be mentioned that solvent-free esterifications of lutein, illustrated by 7 liquid anhydrides (acetic, propionic, 2,2-dimethylpropionic, valeric, pent-4-enoic, decanoic, and oleic) gave good diester yields in the range 78% - 95% when using molar ratio of lutein : acid anhydride : DMAP = 1 : 15 : 30 (Fig. 8A). Only highly reactive liquid pentafluoropropionic

anhydride causes xanthophyll degradation during solvent-free esterification. What is even more intriguing, solid-state reactions carried out with 3 solid anhydrides (phthalic, palmitic, and benzoic) were successful with high lutein diester yields (47%-81%) which were obtained after only 20 min at ambient temperature (Fig. 8B). When scaled up to the industrial scale, these two options should certainly represent viable green approaches. However, here we decided to move forward with β-pinene as the green reaction solvent only to allow for a nonbiased performance comparison for all xanthophyll/acid anhydride reactions described in the invention.

**2.2. Synthesis of xanthophyll esters**

To demonstrate the broad applicability of the developed synthetic platform and to critically evaluate its performance, we prepared, purified, and characterized 55 different xanthophyll esters (from 5 xanthophylls and 11 acid anhydrides). Reactions were carried out under optimised conditions determined above - in β-pinene at ambient temperature and in the presence of DMAP, only the molar ratio of the reagents had to be adjusted. Reaction performance indicators and product-related UV-VIS and MS data are compiled in Table 2. Reactions with all studied xanthophylls (capsanthin, β-cryptoxanthin, lutein, zeaxanthin, and violaxanthin) proceeded in a very similar fashion, giving comparable results.

Overall, esterification reactions were a success with reaction yields higher than 81% (median = 92%), conversions higher than 97% (median = 100%), and selectivities greater than 93% (median = 97%), but there were some obvious outliers (Table 2). Preparation of xanthophyll 2,2-dimethylpropanoates (average yield = 86%) and pentafluoropropanoates (average yield = 32%) demanded substantially higher amounts of acid anhydride and catalyst (up to 500 eq) to drive the reaction forward. Decreased reactivity of 2,2-dimethylpropionic anhydride could be ascribed to steric hindrances imposed by methyl groups, which restrict the nucleophilic attack on the carbonyl, whereas the effects responsible for the decreased yields in the case of pentafluoropropionic anhydride were certainly of electronic nature. Pentafluoropropionic acid has an estimated pKa of 1.37,[54] making its acid anhydride very reactive (labile) towards even trace amounts of moisture. Pentafluoropropanoate is also a good leaving group so the xanthophyll esters in question are more liable to hydrolysis compared to other prepared products and the released acid could induce further product degradation. This instability was indeed observed with all xanthophyll pentafluoropropanoates (average $t_{1/2} \approx 360$ min at ambient temperature, in the dark). Reactions of all 5 studied xanthophylls with phthalic anhydride showed poor conversions (45%-100%) and gave only 19%-41% of the expected diesters. In the presence of DMAP, phthalic anhydride readily precipitated from the reaction mixture and only slowly re-dissolved with the concomitant product formation in the steady-state. To the best of our knowledge, xanthophyll pentafluoropropanoates are reported here for the first time and only a single paper describes an attempt to prepare xanthophyll phthalates, but without success.[20] Many other products were prepared here for the first time as well. Albeit known synthetic pathways to different xanthophyll esters might enable good product yields in some cases,[27,28] none of them can be considered environmentally friendly and economically viable as they all employ toxic reaction solvents derived from non-renewable petrochemical sources and are focused on a particular product[16,17] or a particular class of products.[18,27,37]

Another advantage of the developed synthetic platform is the formation of no or low amounts of unwanted side products (total side products <4.5% on average, Table 2). These were mainly: (i) xanthophyll monoesters (ME) (in the case of dihydroxyxanthophylls), (ii) Z-xanthophyll monoesters (ZME), and (iii) Z-xanthophyll diesters (ZDE).[56] We observed a slightly higher isomerization rate of all-E-products into Z-isomers when unsaturated anhydrides were used (e.g. valeric *versus* pent-4-enoic anhydride and oleic *versus* palmitic anhydride). An important benefit of our mild experimental conditions is also the conservation of labile epoxide moieties[57] during synthesis, which was demonstrated in the case of violaxanthin esterification and supported by MS, UV, and HPTLC data for all 11 violaxanthin esters. Exposure of violaxanthin diesters to HCl vapours clearly demonstrated the presence of two intact epoxide functional groups[8,58] as the colour of the HPTLC bands shifted from yellow to greenish blue (Fig. 9). Published studies rarely bother to report on the formation and quantity of side products;[39] a xanthophyll diester in all-*E*-configuration is generally assumed as a single product (or an all-E monoester in the case of monohydroxyxanthophylls).[17,20,55,59] Only in certain regioselective syntheses where monoesters of dihydroxyxanthophylls were targeted were the relations between the main and side products explicitly given.[16,25,37,38]

After the reaction was terminated we isolated the synthesized xanthophyll esters from the reaction mixture and further purified them. Two independent, simple, and green procedures were developed, evaluated, and compared (Table 2): liquid-liquid extraction (LLE) and solid phase extraction (SPE). During LLE purification, the reaction mixture was washed three consecutive times with 10% $NaHCO_{3(aq)}$ to extract the impurities. A slightly alkaline aqueous solution was best at simultaneously removing excess DMAP, acid anhydrides, and their corresponding acids, but water could also readily be used, giving only 2% lower purities on average. The recoveries after LLE purification were excellent (a yield drop of 4% on average), but, although DMAP was quantitatively removed each time, this strategy was only successful in eliminating relatively hydrophilic, low molecular-weight anhydrides which efficiently partition into the aqueous phase (Table 2). By using SPE, synthesized xanthophyll esters were obtained with a minimum of 74% purity (median 96%), only for phthalates the purity ranged from 42%-96%. SPE purification on a C18 adsorbent employed green solvents in

all stages of the SPE procedure (water, EtOH, isopropanol, acetone, EtOAc) while the use of halogenated and aromatic hydrocarbons was avoided. Compared to LLE, product losses amounted to 9% on average, but this was not surprising due to multiplicity of stages in SPE. All in all, both LLE and SPE could be used to purify a particular xanthophyll ester. LLE approach is greener, faster, simpler, and less expensive, but its use is limited. On the other hand, SPE is truly universal and offers products with high degrees of purity, but slightly larger product loses are encountered during the purification and more waste is created (SPE cartridges, organic solvents).

If not omitted entirely,[16,40,42] conventional purifications of xanthophyll esters involve re-crystallization,[13,14,20,28] TLC purification,[25] or column chromatography.[17,59] These approaches are usually complex, time-consuming, require high energy input, and utilize toxic solvents. Re-crystallization from MeOH, EtOH, and benzene, carried out at elevated temperatures,[13,14,22,28,60] is energy-demanding and causes appreciable loss of xanthophyll esters due to thermal degradation (greater than 14%). Thus, xanthophyll esters are most commonly purified by LLE under acidic or alkaline conditions,[25,36-38,60] but product purities and recoveries after such a treatment are usually not specified[36,37] and in some cases a subsequent TLC purification is applied, accompanied by further product loss and formation of waste.[25,38] Synthetic products were also fractionated on bare silica and RP based stationary phase by means of column chromatography,[17,59] where large amounts of hazardous solvents (hexane : EtOAc or TBME : MeOH : water) were used. Product recovery and purity were not specified in those cases either.

**Table 2.** A collection of data on yields and purities of synthetic products before and after purification. Xanthophyll conversions and reaction selectivities are also listed along with absorption and MS data for all obtained products.

| Capsanthin esters | | | | | | | | | | | |
|---|---|---|---|---|---|---|---|---|---|---|---|
| | **Reaction** | | | | **After LLE** | | **After SPE** | | **Product spectral characteristics** | | |
| | **Yield** | **Purity** | **Conversion** | **Selectivity** | **Yield** | **Purity** | **Yield** | **Purity** | **$\lambda_{max}$ [nm]** | **MS [m/z]** | **MS/MS [m/z]** |
| **diacetate** | 95% (+4% ZDE) | 2% | 100% | 96% | 94% (+2% ZDE) | 98% | 83% (+3% ZDE) | 97% | **471** | 669, 627, **609** | [669]: 627, **609** |
| **dipropanoate** | 96% (+4% ZDE) | 2% | 100% | 96% | 96% (+3% ZDE) | 97% | 72% (+4% <ZDE) | 95% | **471** | 697, 641, **623,** 566 | [697]: **623** [641]: 623, **566** |
| **di(2,2-dimethylpropanoate)** | 79% (12% ZDE; +4% ME) | <1% | 100% | 83% | 79% (+2% ZDE; +7% ME) | 1% | 71% (+7% ZDE; +5% ME) | 86% | **471** | 753, 669, **651**, 585 | [753]: 669, **651** [669]: **651**, 586 |
| **divalerate** | 96% (3% ZDE) | 2% | 100% | 97% | 95% (2% ZDE) | 98% | 82% (4% ZDE) | 96% | **471** | 753, 669, **651**, 585, 566 | [753]: 669, 651 [669]: **651,** 586 |
| **di(pent-4-enoate)** | 86% (8% ZDE; +1% ME) | 1% | 100% | 91% | 85% (2% ZDE; +1% ME) | 96% | 80% (2% ZDE) | 98% | **471** | 749, 667, **649** | [749]: **649,** 667 [667]: 649 |

(continued)

| | Capsanthin esters | | | | | | | | | | |
|---|---|---|---|---|---|---|---|---|---|---|---|
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **dibenzoate** | 93% (+3% ZDE) | 3% | 100% | 97% | 91% (+3% ME) | 6% | 80% (+4% ZDE) | 96% | **471** | 793, 689, **671**, 585 | [793]: **671**, 689 [689]: 671, 566 |
| **didecanoate** | 90% (+4% ZDE) | 2% | 100% | 96% | 90% (+2% ZDE) | 6% | 76% (+1% ZDE) | 99% | **469** | 893, 739, **721** | [893]: 739, **721** [739]: **721** |
| **dipalmitate** | 87% (+1% ZDE) | 2% | 100% | 99% | 86% (+1% ZDE) | 6% | 75% (+1% ZDE) | 79% | **468** | 1062, **805**, 823, 805 | [1062]: **805,** 823 [823]: **805** |
| **dioleate** | 92% (+2% ZDE) | 2% | 100% | 98% | 83% (+2% ZDE) | 5% | 75% (+3% ZDE) | 75% | **468** | 1113, 349, **831** | [1113]: **831**, 849 [849]: **831,** 585 |
| **dipentafluoro-propanoate** | 26% (+8% ZDE; +1% ME) | <1% | 100% | 75% | 26% (+8% ZDE; +1% ME) | <1% | 20% (+4% ZDE) | 96% | **473** | **877**, 731, 713 | [877]: **713** |
| **diphthalate** | 20% (+18% ME) | <1% | 100% | 42% | 13% (+15% ME) | 13% | 10% (+4% ME) | 96% | **471** | 881, 733, **715** | [881]: 733, **715** |

(continued)

| | β-cryptoxanthin esters | | | | | | | | | | |
|---|---|---|---|---|---|---|---|---|---|---|---|
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **acetate** | 94% (+5% ZME) | 2% | 100% | 95% | 92% (+2% ZME) | 98% | 88% (+1% ZME) | 99% | 430, **453,** 478 | **595**, 553, 535 | [595]: 553, **535** |
| **propanoate** | 93% (+4% ZME) | 2% | 100% | 96% | 90% (+2% ZME) | 98% | 85% (+2% ZME) | 98% | 430, **452,** 477 | **609**, 553, 535, 479 | [609]: 553, **535**, 479 |
| **2,2-dimethylpropanoate)** | 81% (+2% ZME) | <1% | 96% | 98% | 78% (+1% ZME) | <1% | 80% (+1% ME) | 99% | 430, **452**, 477 | **637**, 553, 535 | [637]: 553, **535** |
| **valerate** | 92% (+4% ZME) | 2% | 100% | 96% | 84% (+2% ZME) | 98% | 82% (+3% ZME) | 97% | 430, **452**, 477 | **637**, 553, 535 | [637]: 553, **535** |
| **pent-4-enoate** | 86% (+6% ZME) | 2% | 100% | 93% | 84% (+3% ZME) | 97% | 79% (+4% ZME) | 96% | 430, **453**, 477 | **635**, 553, 535 | [635]: 553, **535** |
| **benzoate** | 90% (+4% ZME) | 3% | 100% | 96% | 87% (+2% ZME) | 7% | 86% (+5% ZME) | 95% | 427, **452**, 477 | **657**, 535, 507 | [657]: **535** [657, 535]: **507** |
| **decanoate** | 95% (+4% ZME) | 2% | 100% | 96% | 91% (+2% ZME) | 5% | 84% (+6% ZME) | 94% | 430, **452**, 477 | **707**, 553, 535 | [707]: 553, **535** |
| **palmitate** | 90% (+3% ZME) | 3% | 100% | 97% | 79% (+2% ZME) | 6% | 81% (+5% ZME) | 83% | 430, **452**, 477 | **791**, 353, 553, 443 | [791]: 553, **535**, 443, 353 |

(continued)

| β-cryptoxanthin esters | | | | | | | | | | | |
|---|---|---|---|---|---|---|---|---|---|---|---|
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **oleate** | 95% (+3% ZME) | 2% | 100% | 97% | 82% (+1% ZME) | 7% | 86% (+1% ZME) | 86% | 430, **452**, 477 | **817**, 553, 535 | [817]: 553, **535** |
| **pentafluoropropanoate** | 34% | <1% | 85% | 100% | 29% | <1% | 28% (+6% ZME) | 94% | 427, **452**, 477 | **699**, 535, 523 | [699]: 553, **535,** 523 |
| **phthalate** | 41% | <1% | 84% | 100% | 33% | 9% | 20% | 55% | 430, **454**, 478 | **701**,553,535 | [701]: 553, **535** |
| **Lutein esters** | | | | | | | | | | | |
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **diacetate** | 98% (+2% ME) | 2% | 100% | 98% | 94% (+1% ME) | 99% | 90% (+1% ME) | 99% | 425, **446**, 472 551 | 653, 611, **593**, 533, | [653]: **593**, 533 [611]: **551** |
| **dipropanoate** | >99% | 2% | 100% | 100% | 93% (+1% ME) | 99% | 85% (+1% ME) | 99% | 425, **446**, 471 | 681, **607**, 625 | [681]: **607,** 625 |
| **di(2,2-dimethylpropa noate)** | 96% (+4% ME) | <1% | 100% | 96% | 92% (+2% ME) | <1% | 77% (+2% ME) | 93% | 424, **446,** 472 | 737, 653, **635**, 533 | [737]: 653, **635**, 533 [653]: 635, **551** |

(continued)

| Lutein esters | | | | | | | | | | | |
|---|---|---|---|---|---|---|---|---|---|---|---|
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **divalerate** | 90% | 2% | 100% | 100% | 87% | 99% | 77% (+1% ME) | 99% | 423, **446**, 474 | 737, **635**, 653, 533 | [737]: **635**, 533 |
| **di(pent-4-enoate)** | 92% (+4% ME) | 2% | 100% | 96% | 90% (+3% ME) | 97% | 83% (+1% ME) | 99% | 424, **446**, 472 | 733, **633**, 533 | [733]: **633**, 533 |
| **dibenzoate** | 96% (+3% ME) | 5% | 100% | 97% | 96% (+2% ME) | 8% | 85% (+1% ME; +1% ZDE) | 98% | 423, **446**, 474 | 777, 673, **655**, 533 | [777]: **655**, 533 [673]: **655**, 533 |
| **didecanoate** | 90% (+1% ZDE) | 2% | 100% | 99% | 87% (+1% ZDE) | 7% | 78% | 99% | 424, **446**, 473 | 877, 723, **705**, 551 | [877]: 723, **705** [723]: 705, **551** |
| **dipalmitate** | 88% (+1% ZDE) | 3% | 100% | 99% | 82% | 7% | 80% (+1% ME) | 74% | 424, **445**, 471 | 1045, **789**, 533 | [1045]: **789**, 533 |
| **dioleate** | 96% | 2% | 100% | 100% | 89% | 7% | 84% (+1% ZDE) | 80% | 424, **445**, 472 | 1097, **815**, 533 | [1097]: **815** [1097, 815]: **533** |

(continued)

| Lutein esters | | | | | | | | | | | |
|---|---|---|---|---|---|---|---|---|---|---|---|
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **dipentafluoro-propanoate** | 34% (+4% ME) | <1% | 100% | 85% | 32% (+9% ME) | <1% | 25% (+3% ME) | 97% | 424, **446**, 473 | 861, 715, **697**, 551 | [861]: **697**, 715 [715]: **551** |
| **diphthalate** | 22% (+13% ME) | <1% | 63% | 55% | 11% (+15% ME) | 5% | 16% (+12% ME) | 64% | 424, **446**, 473 | 865, **699**, 551 | [865]: 699, **551** |
| **Zeaxanthin esters** | | | | | | | | | | | |
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **diacetate** | 93% (+1% ZDE) | 2% | 100% | 99% | 93% (+1% ME) | 99% | 80% (+1% ME) | 99% | 427, **452**, 478 | 653, 611, **593**, 533 | [653]: **593**, 533 [611]: **551** |
| **dipropanoate** | 98% (+1% ZDE) | 2% | 100% | 99% | 94% (+1% ZDE) | 99% | 80% (+1% ZDE; +1% ME) | 98% | 425, **452**, 478 | 681, 625, **607**, 625 | [681]: **607**, 625 [625]: 607, **551** |
| **di(2,2-dimethylpropa noate)** | 90% (+2% ZDE; +8% ME) | <1% | 100% | 90% | 96% (+3% ME) | <1% | 98% (+4% ME) | 95% | 428, **452**, 478 | 737, 653, **635**, 533 | [737]: **635**, 653, 533 [653]: 635, **551** |

(continued)

| Zeaxanthin esters | | | | | | | | | | | |
|---|---|---|---|---|---|---|---|---|---|---|---|
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **divalerate** | 95% (+0.5% ME) | 3% | 100% | 99% | 87% | 99% | 87% | >99% | 428, **451,** 477 | 737, 653, **635**, 533 | [737]: **635**, 533 [653]: 635, **551** |
| **di(pent-4-enoate)** | 86% (+1% ZDE; +1% ME) | 2% | 100% | 98% | 86% (+1% ZDE) | 99% | 82% (+1% ZDE) | 99% | 427, **452**, 477 | 733, 651, **633**, 551, 533 | [733]: **633,** 551, 533 |
| **dibenzoate** | >99% | 15% | 100% | 100% | 90% (+1% ZDE; +1% ME) | 8% | 81% (+1% ZDE; +1% ME) | 98% | 426, **452**, 478 | 777, 673, **655** [777] **655**, 673 | [673]: 655, **551**, 533 |
| **didecanoate** | 93% (+1% ZDE) | 3% | 100% | 99% | 90% (+1% ZDE) | 6% | 84% (+1% ZDE) | 99% | 428, **451**, 477 | 877, 723, **705**, 551 | [877]: **705,** 723 [723]: 705, **551** |
| **dipalmitate** | 86% (+3% ZDE) | 2% | 100% | 97% | 80% | 7% | 82% (+2% ZDE) | 98% | 428, **450**, 476 | 1045, **789**, 533 | [1045]: **789**, 533 |
| **dioleate** | 82% (+3% ZDE) | 2% | 100% | 96% | 76% (+2% ZDE) | 6% | 81% (+3% ZDE) | 96% | 427, **451**, 476 | 1097, 823, **815**, 533 | [1097]: **815** [823]: **533** |

(continued)

| Zeaxanthin esters | | | | | | | | | | | |
|---|---|---|---|---|---|---|---|---|---|---|---|
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **dipentafluoro-propanoate** | 34% (+4% ZDE) | <1% | 100% | 89% | 31% (+8% ZDE) | <1% | 25% (+1% ZDE) | 98% | **451**, 477 | **861**, 715, 697, 551 | [861]: **697**, 715 [715]: **551** |
| **diphthalate** | 28% (+12% ME) | <1% | 88% | 70% | 25% (+10% ME) | 4% | 24% ( +7% ME) | 82% | 427, **453**, 477 | 865, **717**, 699 | [865]: 783, **699** |
| **Violaxanthin esters** | | | | | | | | | | | |
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **diacetate** | >99% | 2% | 100% | 100% | >99% | >99% | 82% | >99% | 417, **441**, 470 | **685**, 667, 625, 607 | [685]: 667, **625,** 607 |
| **dipropanoate** | >99% | 2% | 100% | 100% | >99% | >99% | ≥99% | >99% | 417, **441**, 470 | **713,** 659, 657, 639, 621 | [713]: **639, 564,** 547 |
| **di(2,2-dimethylpropa noate)** | 80% (5% ZDE; +15% ME) | <1% | 85% | 80% | 79% (+2% ZDE; +7% ME) | >1% | 71% (+7% ZDE; +5% ME) | 76% | 417, **441**, 470 | **769**, 751, 667, 649, 547 | [769]: **667,** 268 |
| **divalerate** | >99% | 2% | 100% | 100% | >99% | >99% | 82% | >99% | 417, **441**, 470 | **769,** 751, 667, 685, 677, 649, 659 | [769]: 667, **565** [685]: **583** |

(continued)

| Violaxanthin esters | | | | | | | | | | | |
|---|---|---|---|---|---|---|---|---|---|---|---|
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **di(pent-4-enoate)** | 97% (2% ZDE) | 2% | 100% | 98% | 97% (1% ZDE;) | 99% | 96% | >99% | 417, **441**, 470 | **765**, 747, 665, 647 | [765]: **664,** 683 [683]: 665, 590, **582** |
| **dibenzoate** | ≥99% | 3% | 100% | 100% | >99% | 8% | >99% | >99% | 417, **441**, 470 | **809**, 791, 700, 687, 699 | [809]: **687**, 565 [705]: **582,** 565 |
| **didecanoate** | >99% | 3% | 100% | 100% | >99% | 7% | 95% | >99% | 417, **441**, 470 | **909,** 891, 737, 719, 799, 817, 547, 601 | [909]: **736** [755]: 736, **582** |
| **dipalmitate** | >99% | 3% | 100% | 100% | 98% | 8% | 93% | 83% | 417, **440**, 469 | **1077,** 1059, 985, 967, 821, 803 | [1077]: **820** |
| **dioleate** | 84% (+7% ZDE) | 2% | 100% | 92% | 82% (+6% ZDE) | 7% | 83% (+4% ZDE) | 90% | 417, **440,** 469 | **1129**, 1111, 865, 847, 565 | [1129]: **865,** 847 [847]: **601** |
| **dipentafluoro-propanoate** | 38% (+6% ZDE) | <1% | 100% | 86% | 34% (+6% ZDE) | <1% | 28% (+6% ZDE) | >99% | 383, **402,** 427 | **893**, 875, 801, 783, | [893]: **729,** 732 |

(continued)

| | Violaxanthin esters | | | | | | | | | | |
|---|---|---|---|---|---|---|---|---|---|---|---|
| | Reaction | | | | After LLE | | After SPE | | Product spectral characteristics | | |
| | Yield | Purity | Conversion | Selectivity | Yield | Purity | Yield | Purity | $\lambda_{max}$ [nm] | MS [m/z] | MS/MS [m/z] |
| **diphthalate** | 19% (+13% ME) | <1% | 45% | 62% | 13% (+15% ME) | 6% | 11% (+4% ME) | 42% | 417, **440**, 469 | **897**, 749, 731 | [897]: 749, **731** |

Most abundant spectral signals are in boldface.
ME = all-*E*-monoester, ZME = *Z*-monoester, ZDE = *Z*-diester,

$Yield = \frac{n_{p(obtained)}}{n_{p(theoretical)}} \cdot 100\%$ ; $Conversion = \frac{n_{xanth(t=0)} - n_{xanth(t)}}{n_{xanth(t=0)}} \cdot 100\%$ ; $Purity = \frac{n_{p(obtained)}}{n_{all_species}} \cdot 100\%$ ; $Selectivity = \frac{n_{p(obtained)}}{n_{p(obtained)} + n_{side_p(obtained)}} \cdot 100\%$ ;

where: $n_{p(obtained)}$ - obtained amount of product; $n_{p(theoretical)}$ - theoretical amount of product; $n_{xonth(t=0)}$ - amount of free xanthophyll at time t=0; $n_{xonth(t)}$ - amount of free xanthophyll at time t; $n_{all_species}$ - amount of all present species, and $n_{side_p(obtained)}$ - obtained amount of all side products. All quantities are expressed in moles.

### 2.3. Esterification of free xanthophylls obtained from renewable plant sources

Following a successful esterification and purification of pure xanthophylls, we wanted to push our methodology even further towards sustainability. Since commercial prices of particular xanthophylls can easily reach a few hundred euros per milligram of compound, we set out to identify potential renewable sources of these pigments. Food waste such as orange, mandarin, banana, lemon, mango, and avocado peels (ripe and unripe) were screened for free xanthophylls (Fig. 10). Lemon peels proved to be a poor source of pigments. Peels of unripe oranges and mandarins contained >76% of xanthophylls in esterified form (mainly violaxanthin esters) and this content further increased as fruits ripened (>84%) (Fig. 10). These results are in line with the literature data.[61,62] On the other hand, mango, avocado, and banana peels contained predominantly free pigments. These were roughly 2-10 fold more abundant in banana and avocado peels than any other peel studied here, a finding that made these two materials good feedstock candidates. Qualitative and quantitative analytical results are summarized in Table 3. The observed contents of xanthophylls in banana peels were in accordance with the reported values,[63] while certain discrepancies were observed for avocado and mango peels.[64-66] These presumably stem from different fruit varieties and distinct cultivating conditions. It was recently shown that green leaves of Japanese knotweed (*Fallopia japonica* Houtt.), a highly invasive alien plant species to Europe and North America, represent another renewable and rich source of lutein, even richer than banana peels.[8] Thus, we used green leaves of Japanese knotweed as a model renewable source of lutein which could be exploited to produce lutein esters via our developed synthetic platform. By analogy, avocado peels were chosen as a model source of epoxide xanthophylls, mainly violaxanthin and antheraxanthin.

**Table 3.** Viable plant sources of free xanthophylls.

| Xanthophyll source | Part of the plant/ fruit | Major free xanthophylls | Content [mg/100 g dry weight]* | Green aspect |
|---|---|---|---|---|
| Japanese knotweed* | Green leaves | Violaxanthin<br>Lutein<br>Zeaxanthin | 2<br>106<br>5 | Invasive alien plant species |
| Banana | Peel | Lutein | 16 | Food waste |
| Mango | Peel | Lutein | 9 | Food waste |
| Avocado | Peel | Violaxanthin<br>Antheraxanthin<br>Lutein | 2.5<br>9<br>9 | Food waste |

* 300 mg of plant material was extracted with β-pinene (10 mL)
** Reference: [8]

#### 2.3.1. Extraction solvent

A search for more efficient and ecological solvents for carotenoid extraction is ongoing.[67,68] Our goal was to streamline the extraction of free xanthophylls from renewable sources by a green approach and to use the obtained extracts without any further treatment within our synthetic platform. Extraction was optimized on green leaves of Japanese knotweed. Three solvents were tested initially: acetone, EtOAc, and β-pinene. Similar carotenoid chromatographic profile was obtained when using acetone and EtOAc (Fig. 11B and Fig. 11C). On the other hand, bio-derived β-pinene showed greater affinity towards non-polar species such as carotenes while 15% less xanthophylls were isolated (Fig. 11A). Since leaves of Japanese knotweed and other waste materials used in the present invention represent renewable feedstock, a sub-par extraction of xanthophylls should be counterbalanced by a smaller risk that β-pinene poses to the human safety and health. As evidenced from HPTLC chromatograms in Fig. 11 and HPTLC densitograms in Fig. 12, the background of β-pinene extract is also appreciably less busy relative to the other two solvents. The advantages and disadvantages of using either EtOAc/acetone or β-pinene as an extraction (and later as a reaction) solvent are summarized in Table 4.

**Table 4.** A comparison of advantages and disadvantages of EtOAc, acetone and β-pinene as extraction and reaction solvents for the esterification of xanthophylls.

| EtOAc or acetone | β-pinene |
|---|---|
| **Advantages** | |
| A green, biodegradable solvent | A green, biodegradable solvent |
| Inexpensive | Inexpensive |
| Easy to recycle by evaporation | Obtained from renewable sources |
| Higher extraction efficiency for xanthophylls | GRAS |
| | Higher extraction selectivity for xanthophylls |
| | Potential positive effects on human health |
| | Flash point above ambient temperature (36 °C) |
| **Disadvantages** | |
| Flammable | Flammable |
| Low flash point (-4 °C EtOAc, -20 °C acetone) | Viscous |
| | Recycling more challenging |

Xanthophylls from green leaves of Japanese knotweed were also extracted by an environmentally friendly supercritical $CO_2$ (sc-$CO_2$) extraction. The obtained sc-$CO_2$ extract was 10-fold more concentrated in xanthophylls (especially lutein) compared to other solid-liquid extractions (SLE) performed here and the background of sc-$CO_2$ extract was comparable to that of β-pinene (Fig. 11 and Fig. 12). Thus, sc-$CO_2$ extraction should also be considered suitable for a green isolation of xanthophylls from natural materials as already previously demonstrated for carotenoids in general.[68]

### 2.3.2. Esterification reaction

Due to poor reaction yields of xanthophyll pentafluoropropanoates and phthalates we decided to use only 9 acid anhydrides for esterification of xanthophylls from (i) β-pinene and (ii) sc-$CO_2$ extracts of Japanese knotweed leaves, and from (iii) β-pinene extract of avocado peels. Esterifications were carried out under the same conditions as with pure compounds, but a higher excess of acid anhydride and DMAP was used to compensate for interferences from other xanthophylls and compounds bearing reactive nucleophilic functional groups. The excess of reagents could readily be reduced by a more rigorous purification of extracts, but this was not attempted here due to a concomitant generation of additional waste. Also, xanthophylls in the extracts were fairly diluted (0.03 mM) so increasing their concentration in the reaction mixture to 0.3-2.3 mM reduces the consumption of reagents as well. More importantly, the heavier background observed for EtOAc extract of Japanese knotweed leaves (Fig. 11 and Fig. 12) had a strong negative impact on the lutein diacetate product yields compared to β-pinene extract and especially compared to sc-$CO_2$ extract (Fig. 13). Thus, β-pinene and sc-$CO_2$ enable lower waste generation, because fewer equivalents of reagents are required for an equally successful esterification.

The results presented in Table 5 demonstrate a successful extension of the synthetic platform to free xanthophylls obtained from crude plant-based extracts. For the sake of simplicity, only the results for diesters based on extract's most abundant xanthophyll are given in each particular case (lutein or antheraxanthin) although other pigments were also present in the reaction mixture. High reaction yields were obtained (median 94%), but small amounts of monoester and Z-diester side products were observed when using decanoic, palmitic, and oleic anhydrides. After LLE or SPE purification, no major loss of diesters was observed (7% on average).

**Table 5**. A collection of data on yields of synthetic products before and after purification. Xanthophyll conversions and reaction selectivities are also listed for all obtained products.

| | β-pinene extract of green Japanese knotweed leaves (results for lutein) | | | | sc-$CO_2$ extract of green Japanese knotweed leaves (results for lutein) | | | | β-pinene extract of avocado peels (results for antheraxanthin) | | | |
|---|---|---|---|---|---|---|---|---|---|---|---|---|
| | Reaction | | | After LLE/SPE | Reaction | | | After LLE/SPE | Reaction | | | After LLE/SPE |
| | Yield | Conversion | Selectivity | Yield | Yield | Conversion | Selectivity | Yield | Yield | Conversion | Selectivity | Yield |
| **diacetate** | 88% | 100% | 100% | 81%(LLE) | 78% | 100% | 100% | 77%(LLE) | 99% | 100% | 100% | 98%(LLE) |
| **dipropanoate** | >99% | 100% | 100% | 97%(LLE) | 88% | 100% | 100% | 86%(LLE) | 99% | 100% | 100% | 98%(LLE) |
| **di(2,2-dimethylpropanoate)** | 96% | 100% | 100% | 86%(SPE) (+2% ME) | 94% | 100% | 100% | 79%(SPE) | 84% (+2 ME) | 95% | 98% | 80%(SPE) (+1 ME) |
| **divalerate** | 94% (+5% ME) | 100% | 95% | 93%(LLE) (+6% ME) | 93% | 100% | 100% | 90%(LLE) | 97% | 100% | 100% | 93%(LLE) |
| **di(pent-4-enoate)** | 95% (+4% ME) | 100% | 96% | 93% (LLE) (+3% ME) | 94% | 100% | 100% | 94%(LLE) | 99% | 100% | 100% | 91% (LLE) |
| **dibenzoate** | 91% (+8% ME) | 100% | 91% | 85%(SPE) (+7% ME) | 92% | 100% | 100% | 90%(SPE) | 95% | 100% | 100% | 86%(SPE) |
| **didecanoate** | 86% (+13% ZDE) | 100% | 88% | 77% (SPE) (+9% ZDE) | 95% (+4% ZDE) | 100% | 96% | 79%(SPE) (+1 ZDE) | 90% (3% ME) | 100% | 97% | 77% (SPE) (+1% ME) |
| **dipalmitate** | >99% | 100% | 100% | 85% (SPE) | 97% (+2% DE) | 100% | 98% | 78% (SPE) | 88% (+4% ME) | 100% | 96% | 84%(SPE) (+2% ME) |
| **dioleate** | 77% (+8% ZDE) | 100% | 91% | 65% (SPE) (+7% ZDE) | 90% (+2% ME; +2% ZDE) | 100% | 96% | 79% (SPE) | 90% (+2% ME) | 100% | 98% | 84% (SPE) (+1 ME) |

## Conclusion

The preparation of bioactive xanthophyll esters from a perspective of green chemistry and sustainability is discussed for the first time. Instead of delivering an elusive and absolute green approach, we present guidelines on how to choose the right parameters at any stage of the synthetic platform to drive the process either towards sustainability or efficiency. High yields of various xanthophyll esters were obtained by carrying out reactions at ambient temperature with acid anhydrides in the presence of DMAP. Despite some evidenced challenges, polymer supported DMAP which enables recycling could also be used. This is the first synthetic approach where as little as 1.5 eq of anhydride was sufficient to reach high product yield, selectivity, and conversion with low formation of side products. Renewable feedstock as a source of free xanthophylls used here enables industrial applications in support of a sustainable and circular economy. What is more, the exhaustion of invasive alien plant species such as Japanese knotweed is actually even encouraged in order to ensure biodiversity within our ecosystem. Utilization of fruit peels as demonstrated here supports zero waste and a reduction in $CO_2$ emissions.

## REFERENCES

1. R. K. Saini and Y. S. Keum, Food Chem., 2018, 240, 90-103.
2. J. Torregrosa-Crespo, Z. Montero, J. L. Fuentes, M. R. Garcia-Galbis, I. Garbayo, C. Vilchez and R. M. Martinez-Espinosa, Mar. Drugs, 2018, 16, 203.
3. J. Zhang, Z. Sun, P. Sun, T. Chen and F. Chen, Food Funct., 2014, 5, 413-425.
4. J. Mares, Annu. Rev. Nutr., 2016, 36, 571-602.
5. K. T. Amorim-Carrilho, A. Cepeda, C. Fente and P. Regal, TrAC - Trends Anal. Chem., 2014, 56, 49-73.
6. A. Z. Mercadante, D. B. Rodrigues, F. C. Petry and L. R. B. Mariutti, Food Res. Int., 2017, 99, 830-850.
7. P. Jahns and A. R. Holzwarth, Biochim. Biophys. Acta - Bioenerg., 2012, 1817, 182-193.
8. V. Metličar, I. Vovk and A. Albreht, Plants, 2019, 8, 384.
9. F. Cardini, G. Bot. Ital., 1983, 117, 75-97.
10. A. Pérez-Gálvez and M. I. Mínguez-Mosquera, Nutr. Res., 2005, 25, 631-640.
11. L. R. B. Mariutti and A. Z. Mercadante, Arch. Biochem. Biophys., 2018, 648, 36-43.
12. V. Nagy, J. Deli and A. Agócs, in Carotenoid Esters in Foods: Physical, Chemical and Biological Properties: Chemical Synthesis of Carotenoid Esters, ed. A. Z. Mercadante, Royal Society of Chemistry, United Kingdom, 2019, 68-108.
13. P. Karrer and S. Ishikawa, Helv. Chim. Acta, 1930, 13, 709-713.
14. P. Karrer and S. Ishikawa, Helv. Chim. Acta, 1930, 13, 1099-1102.
15. D. Bezbradica, M. Crovic, S. Tanaskovic, N. Lukovic, M. Carevic, A. Milivojevic and Z. Knezevic-Jugovic, Curr. Org. Chem., 2017, 21, 104-138.
16. J. A. Lujan-Montelongo, H. L. Mendoza-Figueroa, C. Silva-Cuevas, A. C. Sánchez-Chávez, L. A. Polindara-Garcia, S. Oliveros-Cruz and M. D. Torres-Cardona, Tetrahedron Lett., 2018, 59, 4096-4101.
17. R. Wang, M. Hou, Y. Zhang, J. Ge and Z. Liu, Catalysis Lett, 2015, 145, 995-999.
18. M. D. T. Cordona and G. Rodriguez, CN1639287A, 2003.
19. X. Huanxin, L. Zuozhen, N. Fanghong, S. Yonglei and J. Banghe, CN1312126C, 2005.
20. M. Háda, V. Nagy, A. Takátsy, J. Deli and A. Agócs, Tetrahedron Lett., 2008, 49, 3524-3526.
21. M. Háda, D. Petrovics, V. Nagy, K. Böddi, J. Deli and A. Agócs, Tetrahedron Lett., 2011, 52, 3195-3197.
22. L. Cholnoky, D. Szabó and J. Szabolcs, Justus Liebigs Ann. Chem., 1957, 606, 194-208.
23. K. Bernhard, G. Englert, H. Mayer, R. K. Müller, A. Rüttimann, M. Vecchi, E. Widmer and R. Zell, Helv. Chim. Acta, 1981, 64, 2469-2484.
24. M. Butnariu, J. Ecosyst. Ecography, 2016, 6, 193-212.
25. M. Háda, V. Nagy, G. Gulyás-Fekete, J. Deli and A. Agócs, Helv. Chim. Acta, 2010, 93, 1149-1155.
26. L. W. Levy, R. H. Binnington and A. S. Tabatznik, US6540654, 2003.
27. L. Jindong and T. Zhengguo, CN107338279A, 2017.
28. A. Gloor and S. Werner, WO2003066583A1, 2003.
29. V. V. De Rosso in Carotenoid Esters in Foods: Physical, Chemical and Biological Properties: Extraction and Cleanup of Xanthophyll Esters, ed. A. Z. Mercadante, Royal Society of Chemistry, United Kingdom, 2019, 285-303.
30. https:/lwww.marketsandmarkets.com/Market -Reports/carotenoid-market-158421566.html (18. 11. 2020).
31. N. Nisar, L. Li, S. Lu, N. C. Khin and B. J. Pogson, Mol. Plant, 2015. 8, 68-82.
32. P. E. Bowen, S. M. Herbst-Espinosa, E. A. Hussain and M. Stacewicz-Sapuntzakis, J. Nutr., 2002, 132, 3668-3673.

33. A. Subagio and N. Morita, Food Chem., 2003, 81, 97-102.
34. P. T. Anastas and J. C. Warner, in Green Chemistry: Theory Pract. Oxford University Press, New York, 2000, 1-148.
35. E. Mellado-Ortega and D. Hornero-Méndez, Food Res. Int., 2017, 2, 111.
36. F. Khachik and G. R. Beecher, J. Agric. Food Chem., 1988, 36, 929-937.
37. F. Khachik, G. R. Beecher and W. R. Lusby, J. Agric. Food Chem., 1989, 37, 1465-1473.
38. H. Fukami, K. Namikawa, N. Sugiura-Tomimori, M. Sumida, K. Katano and M. Nakao, J. Oleo Sci., 2006. 55, 653-656.
39. D. A. Frey, E. W. Kataisto, J. L. Ekmanis, S. O'Malley and S. F. Lockwood, Org. Process Res. Dev., 2004, 8, 796-801.
40. H. Shangguan, S. Zhang, X. Li, Q. Zhou, J. Shi, Q. Deng and F. Huang, RSC Adv., 2020, 10, 8949-8957.
41. F. T. Ahmad, D. E. Mather, H. Y. Law, M. Li, S. A. J. Yousif, K. J. Chalmers, R. E. Asenstorfer and D. J. Mares, J. Cereal Sci., 2015, 64, 109-115.
42. M. Hou, R. Wang, X. Wu, Y. Zhang, J. Ge and Z. Liu, Catal. Lett, 2015, 145, 1825-1829.
43. Z. Q. Liu, L. M. Zhou, P. Liu, P. J. Baker, S. S. Liu, Y. P. Xue, M. Xu and Y. G. Zheng, Appl. Microbiol. Biotechnol., 2015, 99, 8891-8902.
44. F. P. Byrne, S. Jin, G. Paggiola, T. H. M. Petchey, J. H. Clark, T. J. Farmer, A. J. Hunt, C. Robert McElroy and J. Sherwood, Sustain. Chem. Process., 2016, 4, 4-30.
45. D. R. Joshi and N. Adhikari, J. Pharm. Res. Int., 2019, 28, 1-18.
46. D. Prat, A. Wells, J. Hayler, H. Sneddon, C. R. McElroy, S. Abou-Shehada and P. J. Dunn, Green Chem., 2016, 18, 288-296.
47. J. H. Clark, A. Hunt, C. Topi, G. Paggiola, and J. Sherwood, in Sustainable Solvents: Perspectives from Research, Business and International Policy: Green Chemistry Concepts and Metrics for Solvent Selection, Green Chemistry, Royal Society of Chemistry, United Kingdom, 2017, 188-234.
48. E. Yara-Varón, A. Selka, A. S. Fabiano-Tixier, M. Balcells, R. Canela-Garayoa, A. Bily, M. Touaibia and F. Chemat, Green Chem., 2016, 18, 6596-6608.
49. B. Salehi, S. Upadhyay, I. E. Orhan, A. K. Jugran, S. L. D. Jayaweera, D. A. Dias, F. Sharopov, Y. Taheri, N. Martins, N. Baghalpour, W. C. Cho and J. Sharifi-Rad, Biomolecules, 2019, 9, 738.
50. J. Cao, L. Chen, M. Li, F. Cao, L. Zhao and E. Su, Green Chem., 2018, 20, 1879-1886.
51. T. Lemaoui, A. S. Darwish, A. Attoui, F. A. Hatab, N. E. H. Hammoudi, Y. Benguerba, L. F. Vega and I. M. Alnashef, Green Chem., 2020, 22, 8511-8530.
52. M. W. Nam, J. Zhao, M. S. Lee, J. H. Jeong and J. Lee, Green Chem., 2015, 17, 1718-1727.
53. S. Hong, Y. Yuan, Q. Yang, L. Chen, J. Deng, W. Chen, H. Lian, J. D. Mota-Morales and H. Liimatainen, Carbohydr. Polym., 2019, 220, 211-218.
54. MarvinSketch (version 20.08), calculation module developed by ChemAxon,http://www.chemaxon.com/products/marvin/marvinsketch/, 2018.
55. M. Háda, V. Nagy, J. Deli and A. Agócs, Molecules, 2012, 17, 5003-5012.
56. J. E. Sanger, Ecology, 1971, 52, 1075-1089.
57. A. Linden, B. Bürgi and C. H. Eugster, Helv. Chim. Acta, 2004, 87, 1254-1269.
58. J. Deli and E. Osz, Arkivoc., 2004, (vii), 150-168.
59. D. E. Breithaupt and A. Bamedi, J. Agric. Food Chem., 2001, 4, 2064-2070.
60. G. Nadolski, A. J. Cardounel, J. L. Zweier and S. F. Lockwood, Bioorganic Med. Chem. Lett., 2006, 16, 775-781.
61. P. E. Lux, R. Carle, L. Zacarías, M. J. Rodrigo, R. M. Schweiggert and C. B. Steingass, J. Agric. Food Chem., 2019, 67, 13164-13175.
62. F. C. Petry and A. Z. Mercadante, J. Agric. Food Chem., 2016, 64, 8207-8224.
63. C. F. Aquino, L. C. C. Salomão, H. M. Pinheiro-Sant'ana, S. M. R. Ribeiro, D. L. De Siqueira and P. R. Cecon, Rev. Ceres, 2018, 65, 217-226.
64. Q. Y. Lu, Y. Zhang, Y. Wang, D. Wang, R. P. Lee, K. Gao, R. Byrns and D. Heber, J. Agric. Food Chem., 2009, 57, 10408-10413.
65. P. Jimenez, P. Garcia, V. Quitral, K. Vasquez, C. Parra-Ruiz, M. Reyes-Farias, D. F. Garcia-Diaz, P. Robert, C. Encina and J. Soto-Covasich, Food Rev. Int., 2020, DOI: 10.1080/87559129.2020.1717520.
66. K. G. Ranganath, K. S. Shivashankara, T. K. Roy, M. R. Dinesh, G. A. Geetha, K. C. G. Pavithra and K. V. Ravishankar, J. Food Sci. Technol., 2018, 55, 4566-4577.
67. C. Mussagy, V. C. Santos-Ebinuma, K. A. Kurnia, A. Dias, P. J. Carvalho, J. A. P. Coutinho and J. F. B. Pereira, Green Chem., 2020, 22, 8478-8494.
68. S. Marzorati, A. Schievano, A. Idà and L. Verotta, Green Chem., 2020, 22, 187-196.

## Claims

1. Method for the preparation of xanthophyll ester, comprising:

   (i) providing at least one xanthophyll comprising at least one hydroxyl (OH) functional group,
   (ii) esterification of the at least one xanthophyll with acid anhydride in the presence of 4-dimethylaminopyridin (DMAP) or polymer-supported DMAP (pDMAP), wherein the esterification is carried out in the absence of solvent or in the presence of a solvent selected from β-pinene, and ethyl acetate (EtOAc),
   wherein the esterification in step (ii) is carried out at a reaction temperature in the range of 18-25°C, and
   (iii) isolating xanthophyll ester from the reaction mixture.

2. The method according to claim 1, wherein the at least one xanthophyll is selected from capsanthin, β-cryptoxanthin, lutein, zeaxanthin and violaxanthin and combinations thereof.

3. The method according to claim 1 or 2, wherein xanthophyll is provided in pure form or as an extract from peels of banana, mango, and/or avocado and/or as an extract from green leaves of Japanese knotweed, Bohemian knotweed or giant knotweed.

4. The method according to claim 3, wherein the extract is provided by extracting peels of banana, mango, and/or avocado and/or green leaves of Japanese knotweed, Bohemian knotweed or giant knotweed using β-pinene, acetone and/or EtOAc or supercritical $CO_2$ as extracting agent, preferably in nitrogen atmosphere and/or under reduced light conditions.

5. The method according to any one of the preceding claims, wherein the acid anhydride is selected from the group consisting of acetic anhydride, propionic anhydride, 2,2-dimethylpropionic anhydride, valeric anhydride, pent-4-enoic anhydride, benzoic anhydride, decanoic anhydride, palmitic anhydride, oleic anhydride, pentafluoropropionic anhydride, and phthalic anhydride.

6. The method according to any one of the preceding claims, wherein the molar ratio of xanthophyll to acid anhydride is at least 1.5 eq. of acid anhydride per xanthophyll OH group, preferably at least 2.5 eq., 3.0 eq., 5.0 eq., or 7.5 eq. of acid anhydride per xanthophyll OH group and not more than 10 eq. of acid anhydride per xanthophyll OH group.

7. The method according to any one of the preceding claims, wherein the molar ratio of acid anhydride : DMAP or pDMAP is in a range of 1 : 1.5 to 1 : 3, in particular about 1 : 2.

8. The method according to any one of the preceding claims, wherein the esterification in step (ii) is carried out at a reaction temperature in the range of 20-25°C.

9. The method according to any one of the preceding claims, wherein the esterification in step (ii) is carried out in nitrogen atmosphere and/or under reduced light conditions.

10. The method according to any one of the preceding claims, wherein the esterification in step (ii) is carried out in β-pinene.

11. The method according to any one of the preceding claims, wherein isolating xanthophyll ester in step (iii) comprises liquid-liquid extraction (LLE) and/or solid phase extraction (SPE), preferably liquid-liquid extraction using water or aqueous $NaHCO_3$.

## Patentansprüche

1. Verfahren zur Herstellung von Xanthophyllester, umfassend:

   (i) Bereitstellen mindestens eines Xanthophylls mit mindestens einer funktionellen Hydroxyl (OH)-Gruppe,
   (ii) Veresterung des mindestens einen Xanthophylls mit Säureanhydrid in Gegenwart von 4-Dimethylaminopyridin (DMAP) oder Polymergestütztem DMAP (pDMAP), wobei die Veresterung in Abwesenheit von Lösungsmittel oder in Gegenwart eines Lösungsmittels durchgeführt wird, das aus β-Pinen und Ethylacetat (EtOAc) ausgewählt ist,

wobei die Veresterung in Schritt (ii) bei einer Reaktionstemperatur im Bereich von 18-25°C durchgeführt wird, und
(iii) Isolieren von Xanthophyllester aus dem Reaktionsgemisch.

**2.** Verfahren nach Anspruch1, wobei das mindestens eine Xanthophyll ausgewählt ist aus Capsanthin, β-Cryptoxanthin, Lutein, Zeaxanthin und Violaxanthin und Kombinationen davon.

**3.** Verfahren nach Anspruch 1 oder 2, wobei Xanthophyll in reiner Form oder als Extrakt aus Schalen von Banane, Mango und/oder Avocado und/oder als Extrakt aus grünen Blättern des Japanischen Staudenknöterichs, des Böhmischen Staudenknöterichs oder des Riesenknöterichs bereitgestellt wird.

**4.** Verfahren nach Anspruch 3, wobei der Extrakt bereitgestellt wird durch Extrahieren von Schalen von Banane, Mango und/oder Avocado und/oder Blättern des Japanischen Staudenknöterichs, des Böhmischen Staudenknöterichs oder des Riesenknöterichs, unter Verwendung von β-Pinen, Aceton und/oder EtOAc oder überkritischem $CO_2$ als Extraktionsmittel, vorzugsweise in Stickstoffatmosphäre und/oder unter reduzierten Lichtverhältnissen.

**5.** Verfahren nach einem der vorhergehenden Ansprüche, wobei das Säureanhydrid ausgewählt ist aus der Gruppe bestehend aus Essigsäureanhydrid, Propionsäureanhydrid, 2,2-Dimethylpropionsäureanhydrid, Valeriansäureanhydrid, Pent-4-ensäureanhydrid, Benzoesäureanhydrid, Decansäureanhydrid, Palmitinsäureanhydrid, Ölsäureanhydrid, Pentafluorpropionsäureanhydrid, und Phthalsäureanhydrid.

**6.** Verfahren nach einem der vorhergehenden Ansprüche, wobei das molare Verhältnis von Xanthophyll zu Säureanhydrid mindestens 1,5 eq. Säureanhydrid pro Xanthophyll OH-Gruppe beträgt, vorzugsweise mindestens 2,5 eq., 3,.0 eq., 5,0 eq. oder 7,5 eq. Säureanhydrid pro Xanthophyll OH-Gruppe und nicht mehr als 10 eq. Säureanhydrid pro Xanthophyll OH-Gruppe.

**7.** Verfahren nach einem der vorhergehenden Ansprüche, wobei das molare Verhältnis von Säureanhydrid : DMAP oder pDMAP im Bereich von 1 : 1.5 bis 1 : 3 liegt, insbesondere bei etwa 1 : 2.

**8.** Verfahren nach einem der vorhergehenden Ansprüche, wobei die Veresterung in Schritt (ii) bei einer Reaktionstemperatur im Bereich von 20-25°C durchgeführt wird.

**9.** Verfahren nach einem der vorhergehenden Ansprüche, wobei die Veresterung in Schritt (ii) in einer Stickstoffatmosphäre und/oder unter reduzierten Lichtverhältnissen durchgeführt wird.

**10.** Verfahren nach einem der vorhergehenden Ansprüche, wobei die Veresterung in Schritt (ii) in β-Pinen durchgeführt wird.

**11.** Verfahren nach einem der vorhergehenden Ansprüche, wobei das Isolieren des Xanthophyllesters in Schritt (iii) flüssig-flüssig Extraktion (LLE) und/oder Festphasenextraktion (SPE) umfasst, vorzugsweise flüssig-flüssig Extraktion unter Verwendung von Wasser oder wässrigem NaHCOs.

## Revendications

**1.** Procédé de préparation d'ester de xanthophylles, comprenant :

(i) la fourniture d'au moins une xanthophylle comprenant au moins un groupe fonctionnel hydroxyle (OH),
(ii) l'estérification de l'au moins une xanthophylle avec de l'anhydride d'acide en présence de 4-diméthylaminopyridine (DMAP) ou de DMAP sur support polymère (pDMAP), dans lequel l'estérification est réalisée en l'absence de solvant ou en présence d'un solvant choisi parmi le β-pinène, et l'acétate d'éthyle (EtOAc), dans lequel l'estérification à l'étape (ii) est réalisée à une température de réaction dans la plage de 18 à 25 °C, et
(iii) l'isolement de l'ester de xanthophylle du mélange réactionnel.

**2.** Procédé selon la revendication 1, dans lequel l'au moins une xanthophylle est choisie parmi la capsanthine, la β-cryptoxanthine, la lutéine, la zéaxanthine et la violaxanthine et les combinaisons de celles-ci.

**3.** Procédé selon la revendication 1 ou la revendication 2, dans lequel la xanthophylle est fournie sous forme pure ou comme un extrait de peaux de banane, de mangue et/ou d'avocat et/ou comme un extrait de feuilles vertes de

renouée du Japon, de renouée de Bohème ou de renouée géante.

4. Procédé selon la revendication 3, dans lequel l'extrait est fourni par extraction à partir de peaux de banane, de mangue et/ou d'avocat et/ou de feuilles vertes de renouée du Japon, de renouée de Bohème ou de renouée géante en utilisant le β-pinène, l'acétone et/ou l'EtOAc ou le $CO_2$ supercritique comme agent d'extraction, de préférence dans une atmosphère d'azote et/ou dans des conditions de lumière réduite.

5. Procédé selon l'une quelconque des revendications précédentes, dans lequel l'anhydride d'acide est choisi dans le groupe constitué par l'anhydride acétique, l'anhydride propionique, l'anhydride 2,2-diméthylpropionique, l'anhydride valérique, l'anhydride pent-4-énoïque, l'anhydride benzoïque, l'anhydride décanoïque, l'anhydride palmitique, l'anhydride oléique, l'anhydride pentafluoropropionique et l'anhydride phtalique.

6. Procédé selon l'une quelconque des revendications précédentes, dans lequel le rapport molaire de xanthophylle à l'anhydride d'acide est au moins 1,5 équ. d'anhydride d'acide par groupe OH de xanthophylle, de préférence au moins 2,5 équ., 3,0 équ., 5,0 équ., ou 7,5 équ. d'anhydride d'acide par groupe OH de xanthophylle et pas plus de 10 équ. d'anhydride d'acide par groupe OH de xanthophylle.

7. Procédé selon l'une quelconque des revendications précédentes, dans lequel le rapport molaire d'anhydride d'acide : DMAP ou pDMAP se situe dans une plage de 1 : 1,5 à 1 : 3, en particulier d'environ 1 : 2.

8. Procédé selon l'une quelconque des revendications précédentes, dans lequel l'estérification à l'étape (ii) est réalisée à une température de réaction dans la plage de 20 à 25 °C.

9. Procédé selon l'une quelconque des revendications précédentes, dans lequel l'estérification à l'étape (ii) est réalisée dans une atmosphère d'azote et/ou dans des conditions de lumière réduite.

10. Procédé selon l'une quelconque des revendications précédentes, dans lequel l'estérification à l'étape (ii) est réalisée dans du β-pinène.

11. Procédé selon l'une quelconque des revendications précédentes, dans lequel l'isolement de l'ester de xanthophylle à l'étape (iii) comprend une extraction liquide-liquide (LLE) et/ou une extraction de phase solide (SPE), de préférence une extraction liquide-liquide utilisant de l'eau ou du $NaHCO_3$ aqueux.

Fig. 1

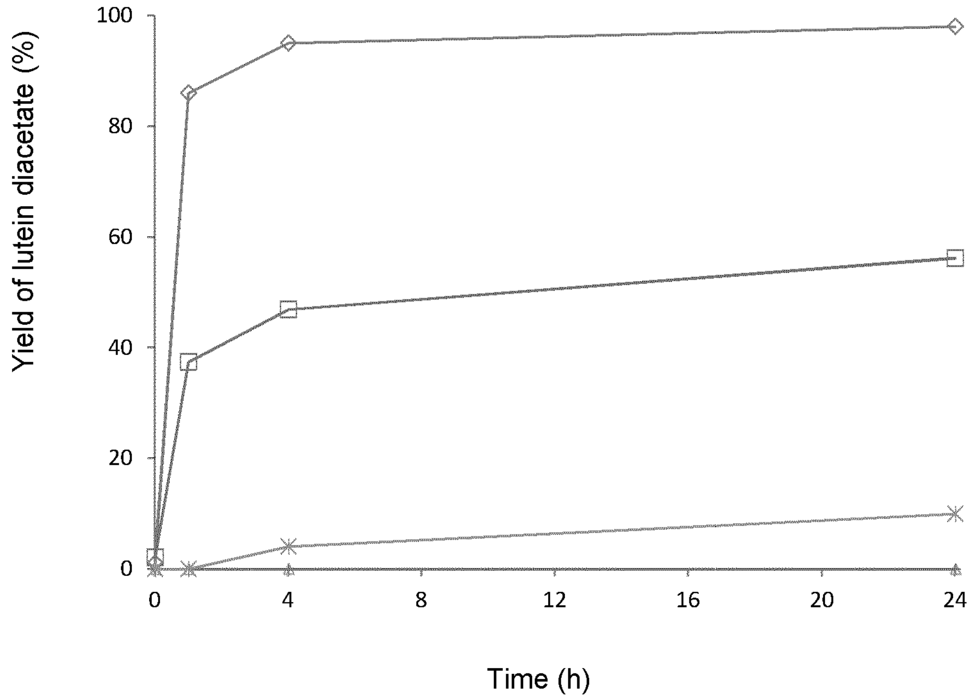
Yield of lutein diacetate (%)
100
80
60
40
20
0
0
4
8
12
16
20
24
Time (h)

Fig. 2

A

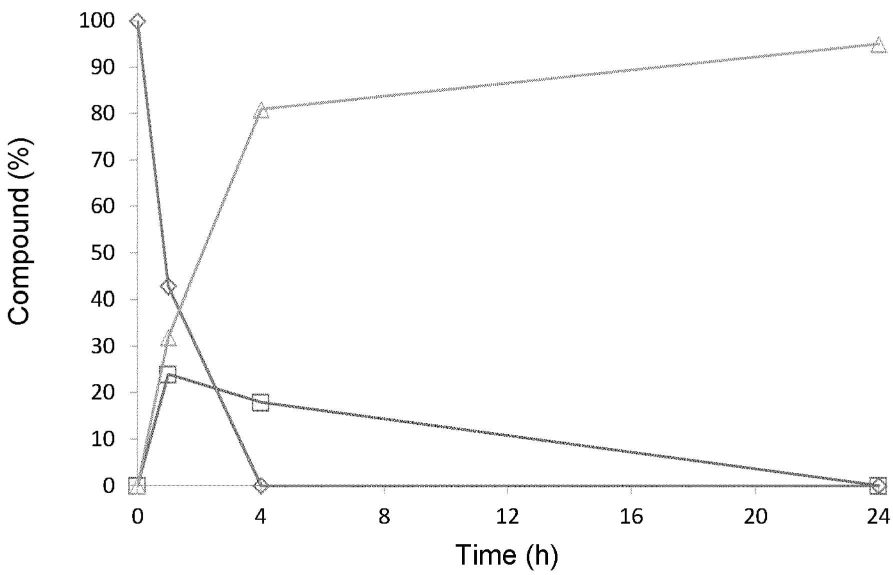
Compound (%)
100
90
80
70
60
50
40
30
20
10
0
0
4
8
12
16
20
24
Time (h)

B

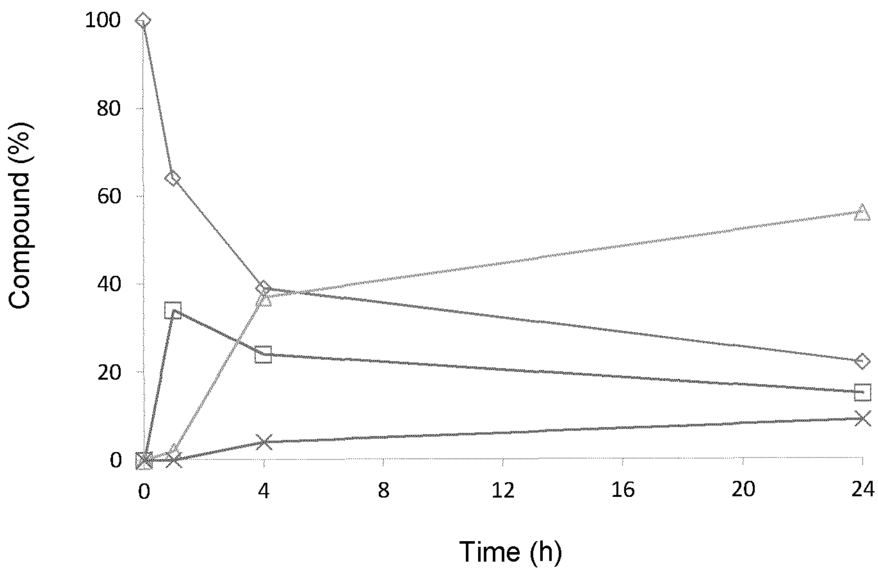

## Fig. 3

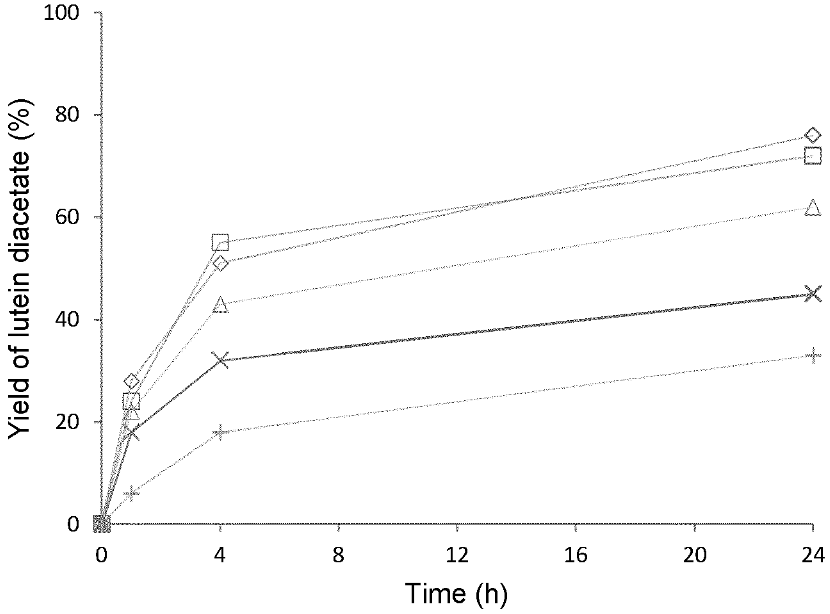
Yield of lutein diacetate (%)
100
80
60
40
20
0
0
4
8
12
16
20
24
Time (h)

## Fig. 4

### A

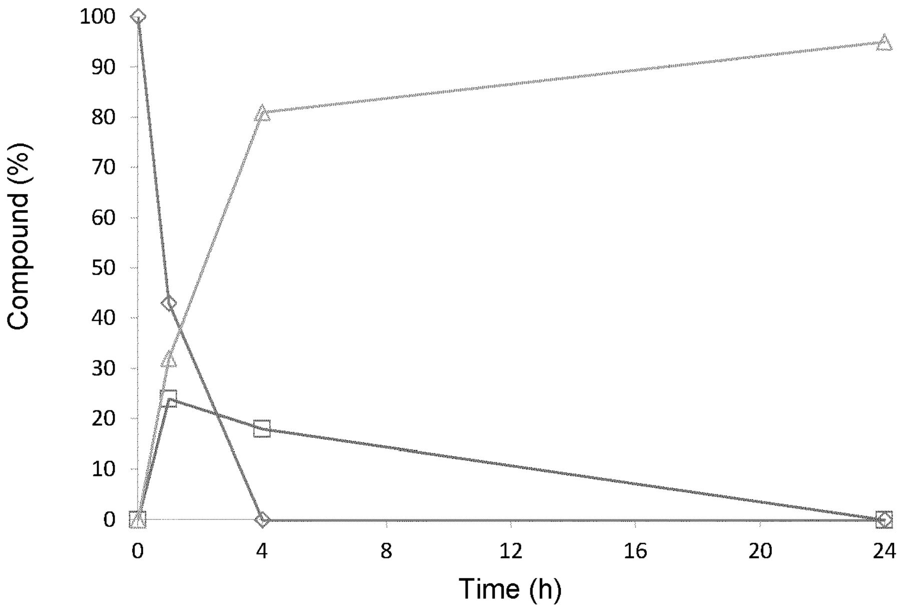
Compound (%)
100
90
80
70
60
50
40
30
20
10
0
0
4
8
12
16
20
24
Time (h)

B

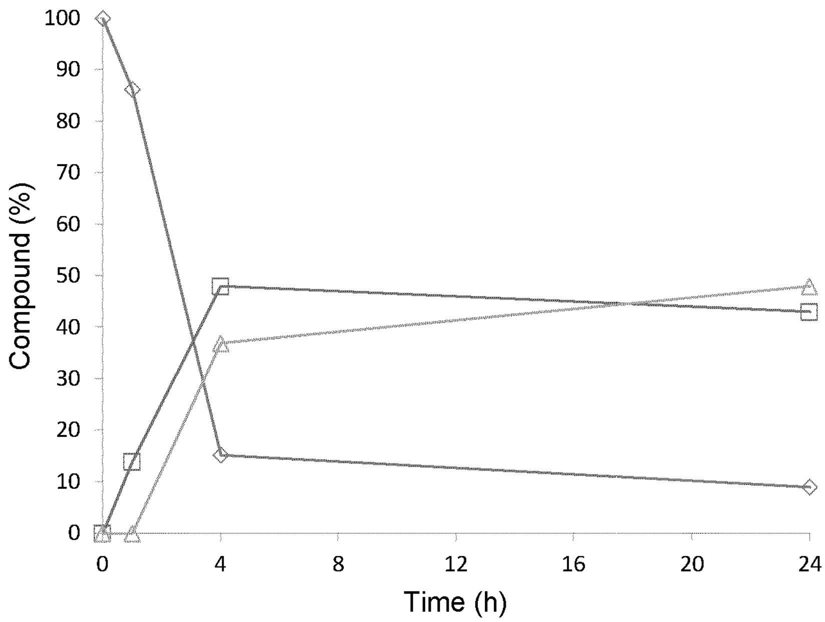
Compound (%)
100
90
80
70
60
50
40
30
20
10
0
0
4
8
12
16
20
24
Time (h)

C

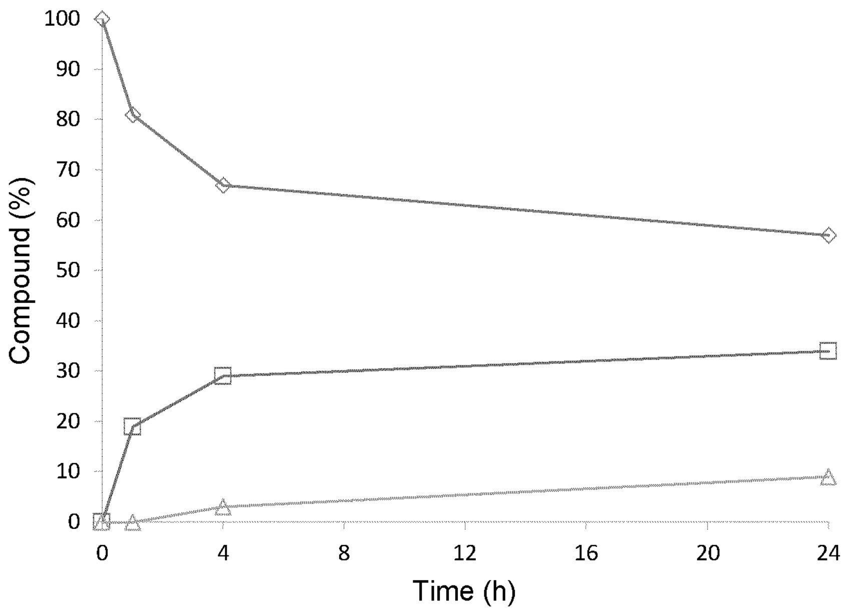
Compound (%)
100
90
80
70
60
50
40
30
20
10
0
0
4
8
12
16
20
24
Time (h)

# Fig. 5

A

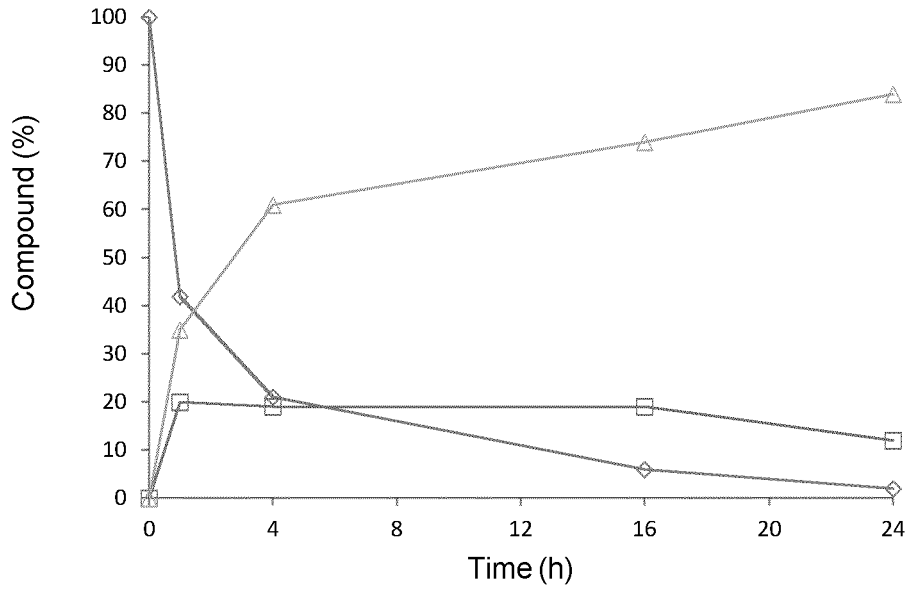
100
90
80
70
60
50
40
30
20
10
0
Compound (%)
0
4
8
12
16
20
24
Time (h)

B

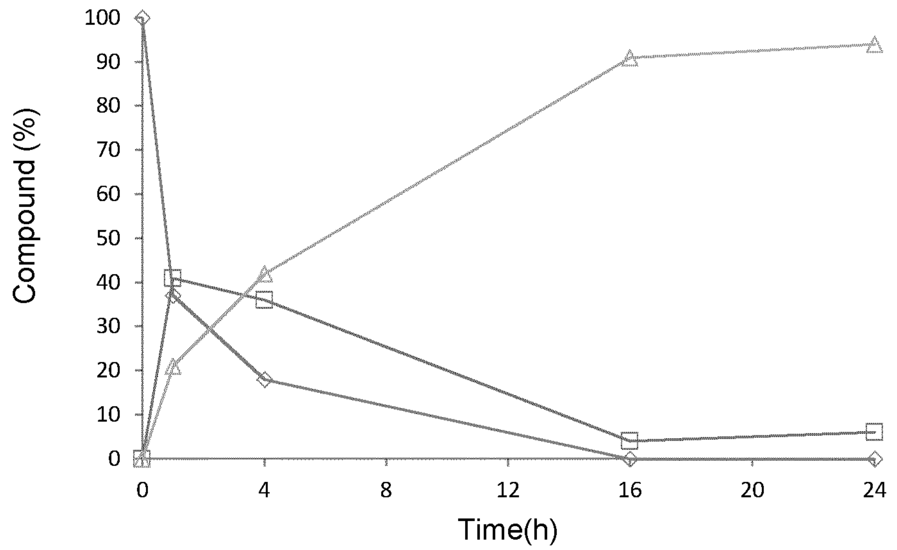
100
90
80
70
60
50
40
30
20
10
0
Compound (%)
0
4
8
12
16
20
24
Time(h)

# Fig. 6

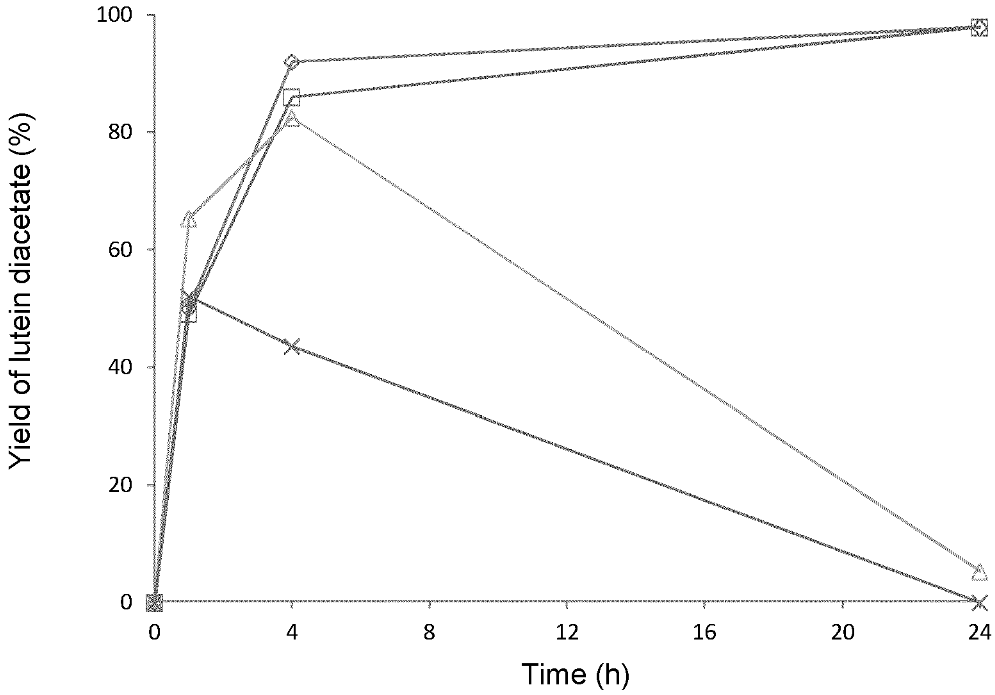
100
80
60
40
20
0
Yield of lutein diacetate (%)
0
4
8
12
16
20
24
Time (h)

**Fig. 7**

**A**

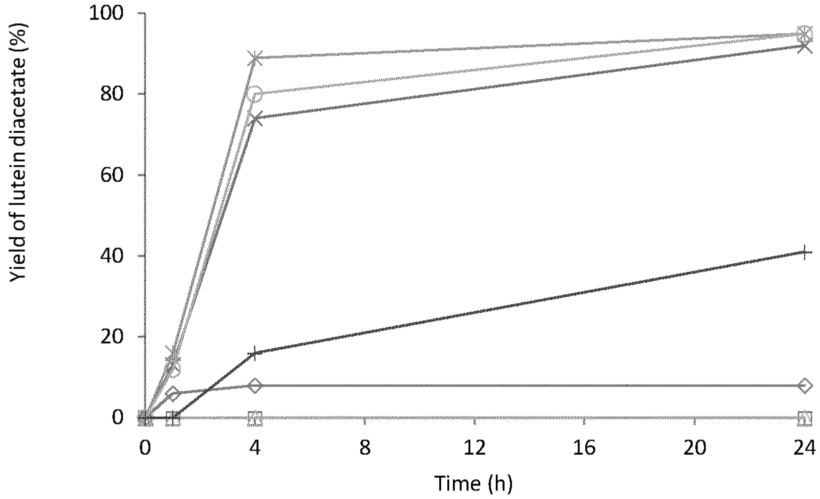
Yield of lutein diacetate (%)
100
80
60
40
20
0
0
4
8
12
16
20
24
Time (h)

**B**

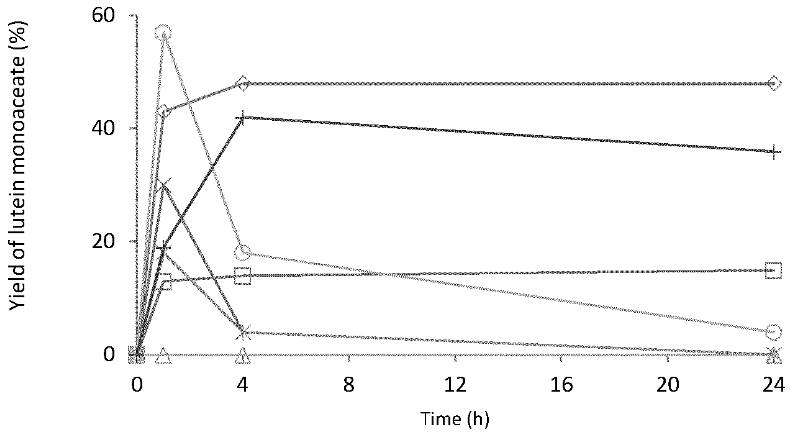
Yield of lutein monoaceate (%)
60
40
20
0
0
4
8
12
16
20
24
Time (h)

**C**

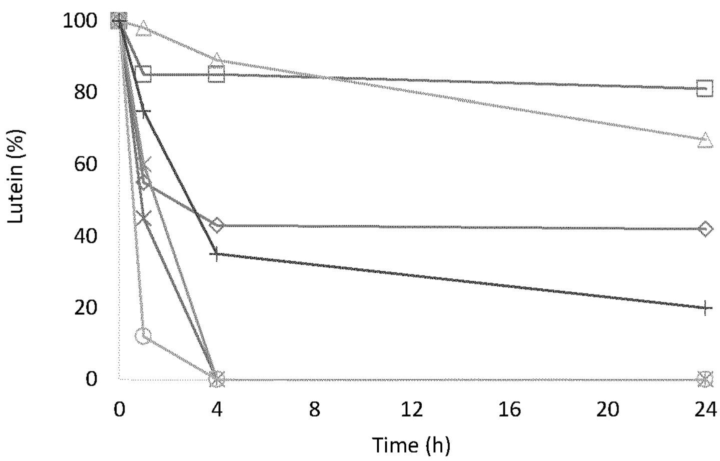
Lutein (%)
100
80
60
40
20
0
0
4
8
12
16
20
24
Time (h)

# Fig. 8

## A

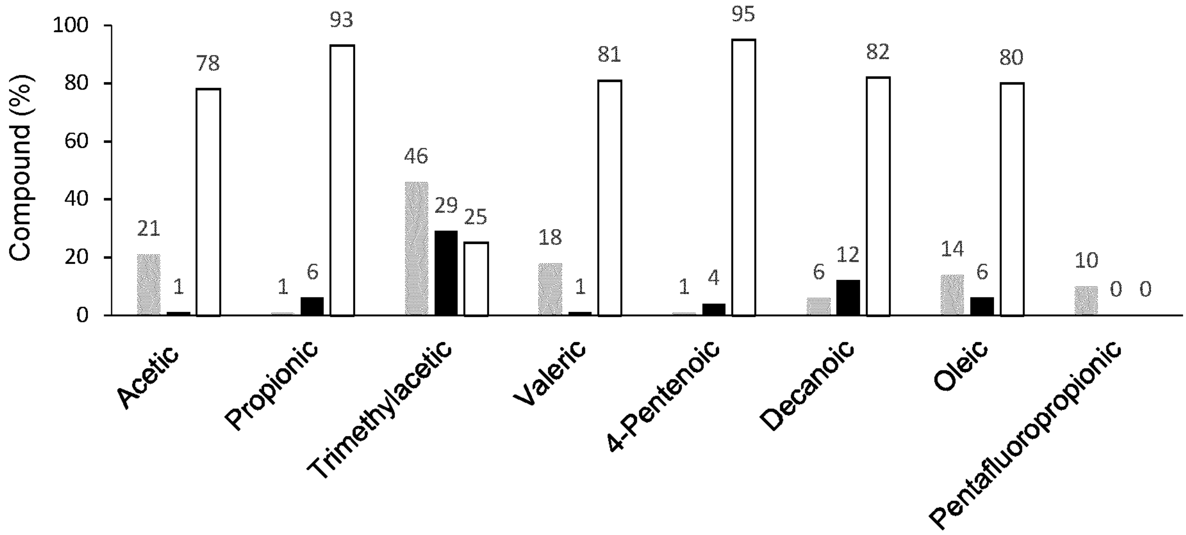
Compound (%)
100
80
60
40
20
0
21
1
78
1
6
93
46
29
25
18
1
81
1
4
95
6
12
82
14
6
80
10
0
0
Acetic
Propionic
Trimethylacetic
Valeric
4-Pentenoic
Decanoic
Oleic
Pentafluoropropionic

## B

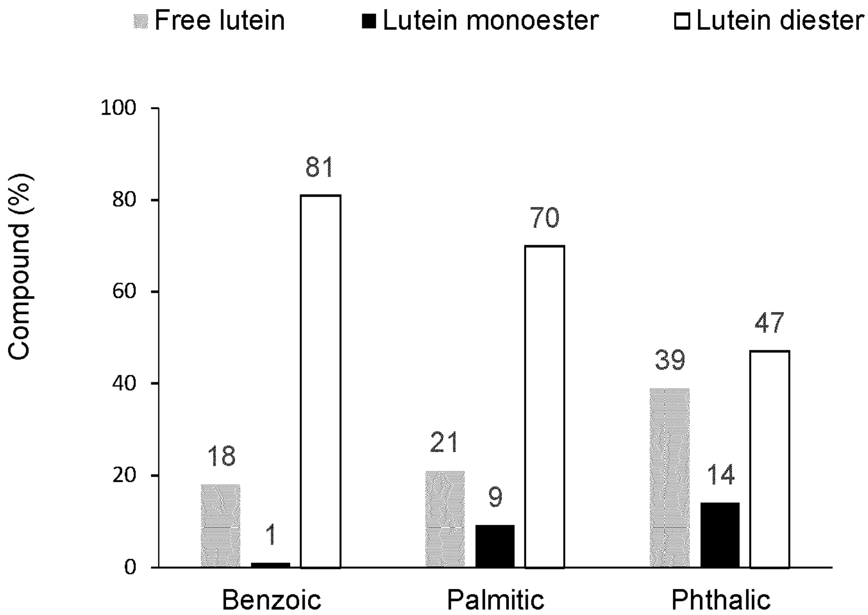
Free lutein
Lutein monoester
Lutein diester
Compound (%)
100
80
60
40
20
0
18
1
81
21
9
70
39
14
47
Benzoic
Palmitic
Phthalic

Fig. 9

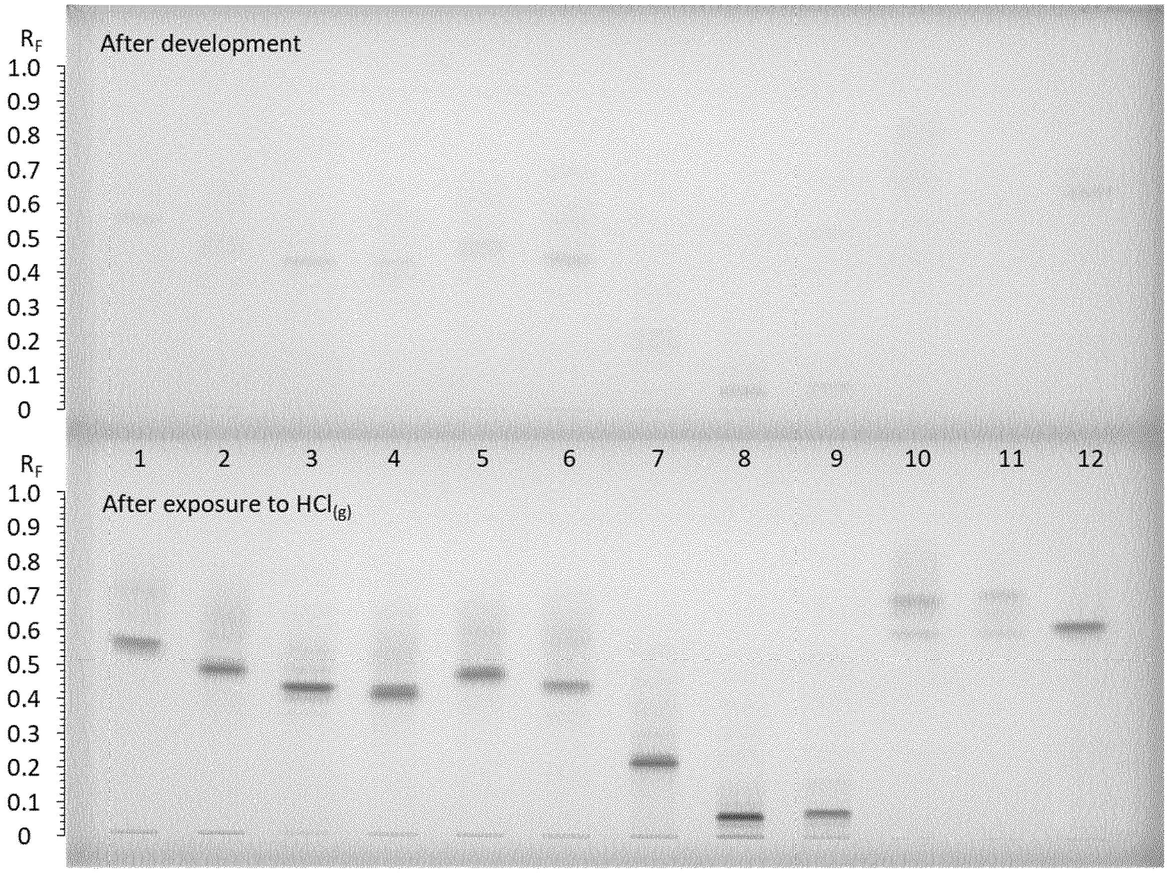

$R_F$
After development
1.0
0.9
0.8
0.7
0.6
0.5
0.4
0.3
0.2
0.1
0
1
2
3
4
5
6
7
8
9
10
11
12
$R_F$
After exposure to $HCl_{(g)}$
1.0
0.9
0.8
0.7
0.6
0.5
0.4
0.3
0.2
0.1
0

# Fig. 10

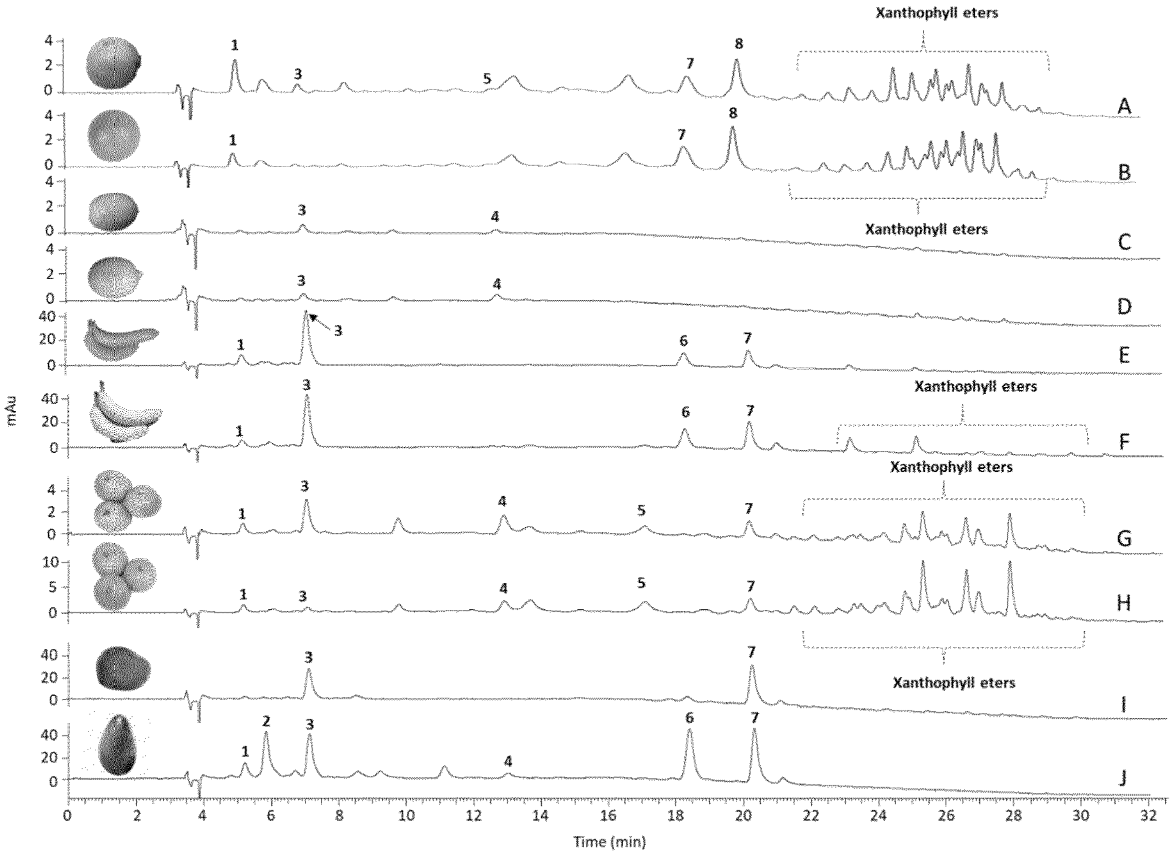

**Fig. 11**

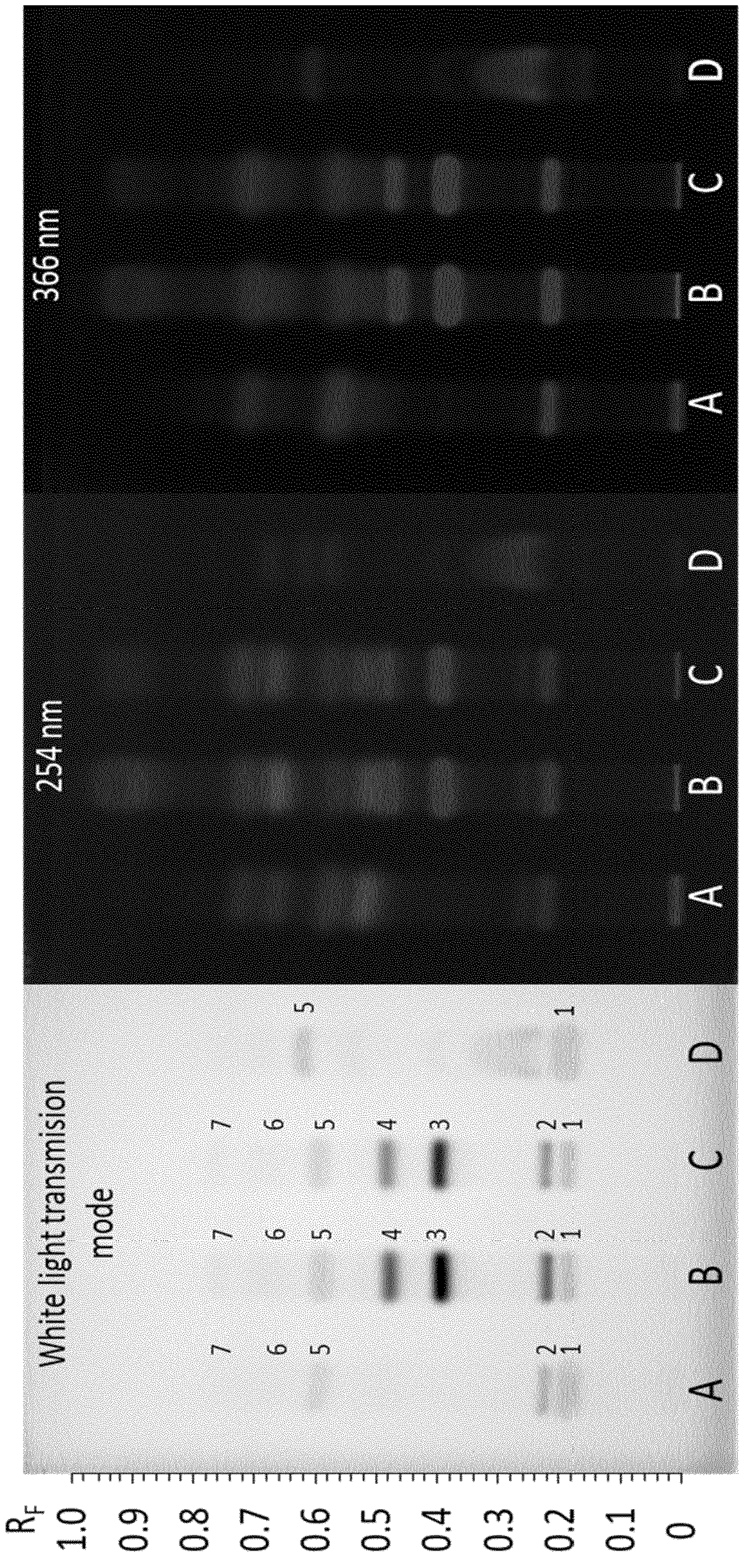
White light transmision mode
254 nm
366 nm
A
B
C
D
$R_F$
1.0
0.9
0.8
0.7
0.6
0.5
0.4
0.3
0.2
0.1
0

Fig. 12

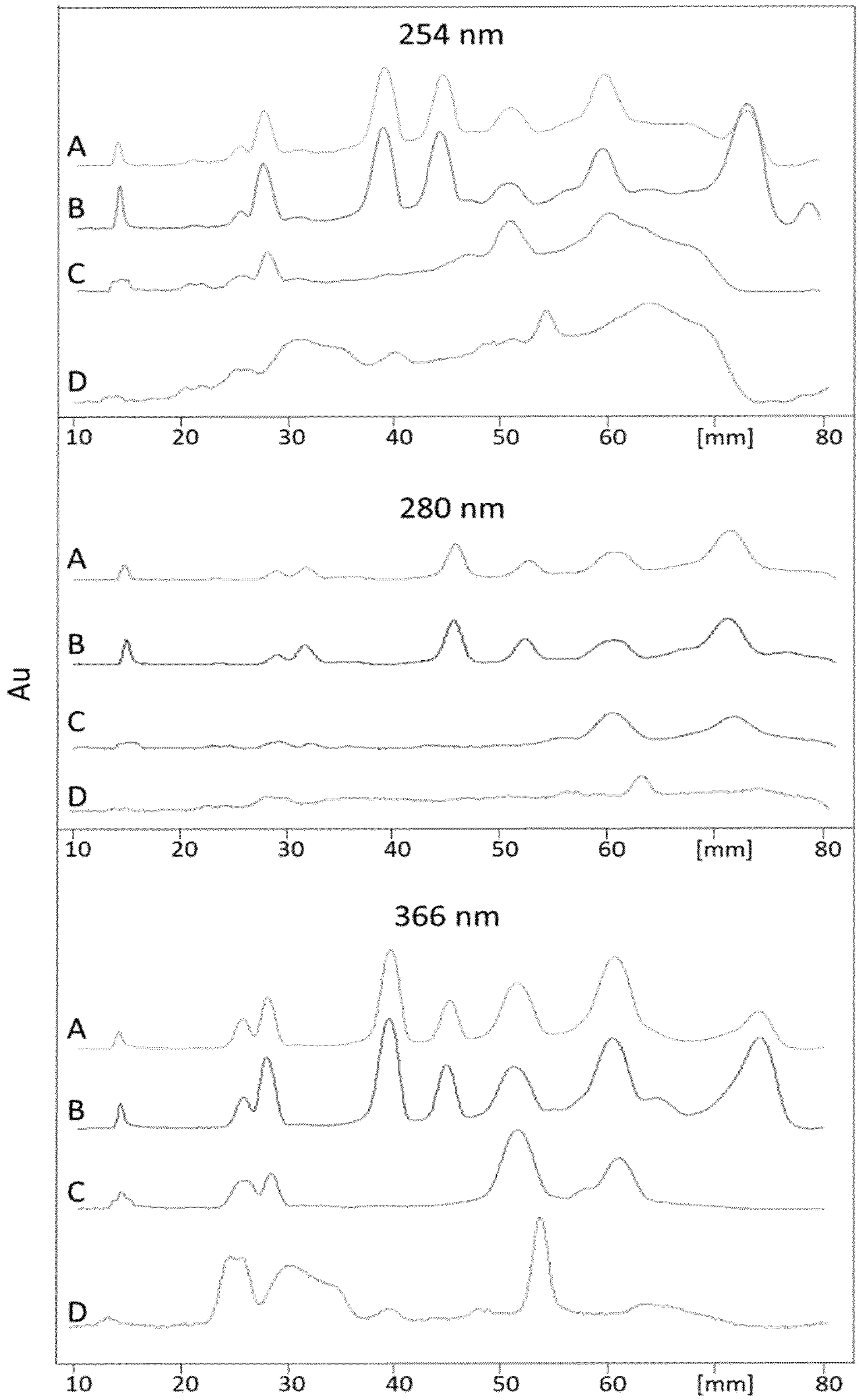
254 nm
A
B
C
D
10 20 30 40 50 60 [mm] 80
280 nm
A
B
C
D
10 20 30 40 50 60 [mm] 80
Au
366 nm
A
B
C
D
10 20 30 40 50 60 [mm] 80

**Fig. 13**

**A**

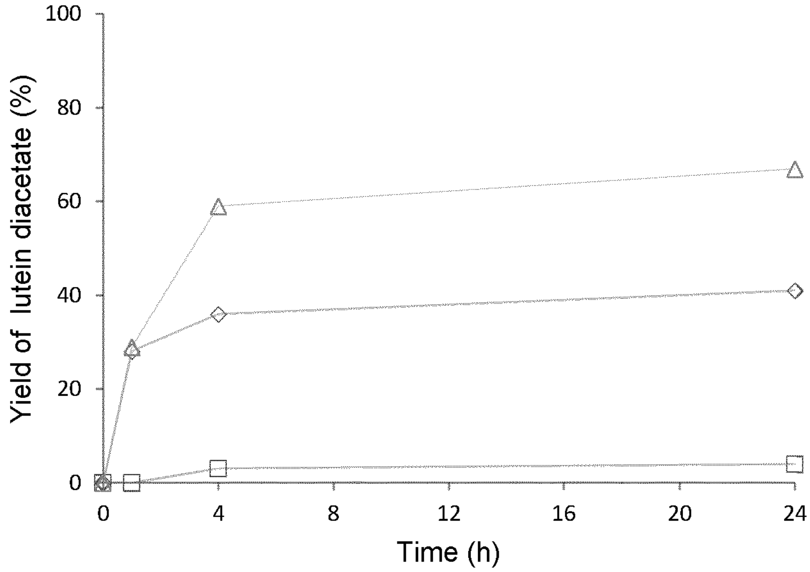
100
80
60
40
20
0
Yield of lutein diacetate (%)
0
4
8
12
16
20
24
Time (h)

**B**

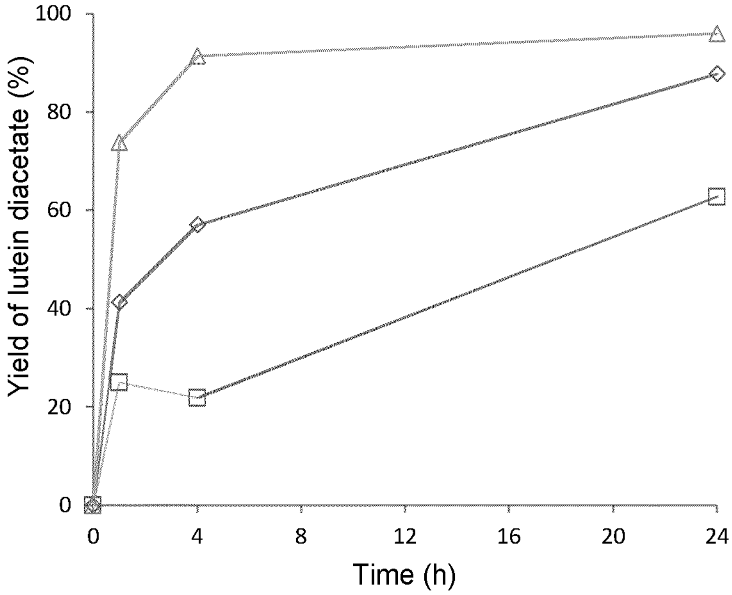
100
80
60
40
20
0
Yield of lutein diacetate (%)
0
4
8
12
16
20
24
Time (h)

C

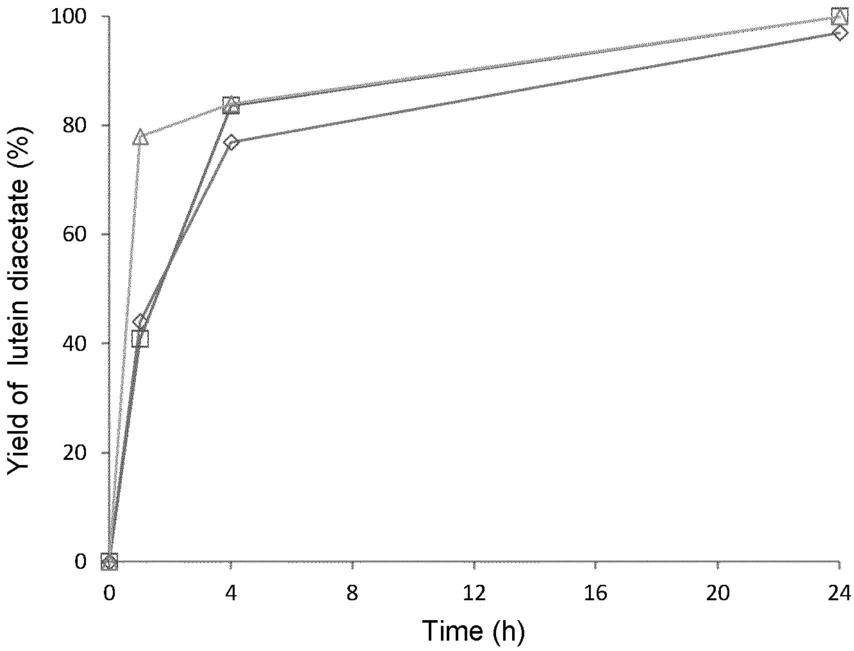
Yield of lutein diacetate (%)
100
80
60
40
20
0
0
4
8
12
16
20
24
Time (h)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03066583 A
- CN 103830113
- CN 1639287 A, M. D. T. Cordona and G. Rodriguez
- CN 1312126 C, X. Huanxin, L. Zuozhen, N. Fanghong, S. Yonglei and J. Banghe
- US 6540654 B, L. W. Levy, R. H. Binnington and A. S. Tabatznik
- CN 107338279 A, L. Jindong and T. Zhengguo,
- WO 2003066583 A1, A. Gloor and S. Werner


### Non-patent literature cited in the description


- **R. K. SAINI ; Y. S. KEUM.** *Food Chem.,* 2018, vol. 240, 90-103
- **J. TORREGROSA-CRESPO ; Z. MONTERO ; J. L. FUENTES ; M. R. GARCIA-GALBIS ; I. GARBAYO ; C. VILCHEZ ; R. M. MARTINEZ-ESPINOSA.** *Mar. Drugs,* 2018, vol. 16, 203
- **J. ZHANG ; Z. SUN ; P. SUN ; T. CHEN ; F. CHEN.** *Food Funct.,* 2014, vol. 5, 413-425
- **J. MARES.** *Annu. Rev. Nutr.,* 2016, vol. 36, 571-602
- **K. T. AMORIM-CARRILHO ; A. CEPEDA ; C. FENTE ; P. REGAL.** *TrAC - Trends Anal. Chem,* 2014, vol. 56, 49-73
- **A. Z. MERCADANTE ; D. B. RODRIGUES ; F. C. PETRY ; L. R. B. MARIUTTI.** *Food Res. Int.,* 2017, vol. 99, 830-850
- **P. JAHNS ; A. R. HOLZWARTH.** *Biochim. Biophys. Acta - Bioenerg.,* 2012, vol. 1817, 182-193
- **V. METLIČAR ; I. VOVK ; A. ALBREHT.** *Plants,* 2019, vol. 8, 384
- **F. CARDINI.** *G. Bot. Ital.,* 1983, vol. 117, 75-97
- **A. PÉREZ-GÁLVEZ ; M. I. MÍNGUEZ-MOSQUERA.** *Nutr. Res,* 2005, vol. 25, 631-640
- **L. R. B. MARIUTTI ; A. Z. MERCADANTE.** *Arch. Biochem. Biophys,* 2018, vol. 648, 36-43
- **V. NAGY ; J. DELI ; A. AGÓCS.** Carotenoid Esters in Foods: Physical, Chemical and Biological Properties: Chemical Synthesis of Carotenoid Esters,. Royal Society of Chemistry, 2019, 68-108
- **P. KARRER ; S. ISHIKAWA.** *Helv. Chim. Acta,* 1930, vol. 13, 709-713
- **P. KARRER ; S. ISHIKAWA.** *Helv. Chim. Acta,* 1930, vol. 13, 1099-1102
- **D. BEZBRADICA ; M. CROVIC ; S. TANASKOVIC ; N. LUKOVIC ; M. CAREVIC ; A. MILIVOJEVIC ; Z. KNEZEVIC-JUGOVIC.** *Curr. Org. Chem,* 2017, vol. 21, 104-138
- **J. A. LUJAN-MONTELONGO ; H. L. MENDOZA-FIGUEROA ; C. SILVA-CUEVAS ; A. C. SÁNCHEZ-CHÁVEZ ; L. A. POLINDARA-GARCIA ; S. OLIVEROS-CRUZ ; M. D. TORRES-CARDONA.** *Tetrahedron Lett.,* 2018, vol. 59, 4096-4101
- **R. WANG ; M. HOU ; Y. ZHANG ; J. GE ; Z. LIU.** *Catalysis Lett,* 2015, vol. 145, 995-999
- **M. HÁDA ; V. NAGY ; A. TAKÁTSY ; J. DELI ; A. AGÓCS.** *Tetrahedron Lett.,* 2008, vol. 49, 3524-3526
- **M. HÁDA ; D. PETROVICS ; V. NAGY ; K. BÖDDI ; J. DELI ; A. AGÓCS.** *Tetrahedron Lett.,* 2011, vol. 52, 3195-3197
- **L. CHOLNOKY ; D. SZABÓ ; J. SZABOLCS.** *Justus Liebigs Ann. Chem,* 1957, vol. 606, 194-208
- **K. BERNHARD ; G. ENGLERT ; H. MAYER ; R. K. MÜLLER ; A. RÜTTIMANN ; M. VECCHI ; E. WIDMER ; R. ZELL.** *Helv. Chim. Acta,* 1981, vol. 64, 2469-2484
- **M. BUTNARIU.** *J. Ecosyst. Ecography,* 2016, vol. 6, 193-212
- **M. HÁDA ; V. NAGY ; G. GULYÁS-FEKETE ; J. DELI ; A. AGÓCS.** *Helv. Chim. Acta,* 2010, vol. 93, 1149-1155
- **V. V. DE ROSSO.** Carotenoid Esters in Foods: Physical, Chemical and Biological Properties: Extraction and Cleanup of Xanthophyll Esters. Royal Society of Chemistry, 2019, 285-303
- **N. NISAR ; L. LI ; S. LU ; N. C. KHIN ; B. J. POGSON.** *Mol. Plant,* 2015, vol. 8, 68-82
- **P. E. BOWEN ; S. M. HERBST-ESPINOSA ; E. A. HUSSAIN ; M. STACEWICZ-SAPUNTZAKIS.** *J. Nutr.,* 2002, vol. 132, 3668-3673
- **A. SUBAGIO ; N. MORITA.** *Food Chem.,* 2003, vol. 81, 97-102
- **P. T. ANASTAS ; J. C. WARNER.** Green Chemistry: Theory Pract. Oxford University Press, 2000, 1-148

- **E. MELLADO-ORTEGA ; D. HORNERO-MÉNDEZ.** *Food Res. Int.,* 2017, vol. 2, 111
- **F. KHACHIK ; G. R. BEECHER.** *J. Agric. Food Chem.,* 1988, vol. 36, 929-937
- **F. KHACHIK ; G. R. BEECHER ; W. R. LUSBY.** *J. Agric. Food Chem.,* 1989, vol. 37, 1465-1473
- **H. FUKAMI ; K. NAMIKAWA ; N. SUGIURA-TOMIMORI ; M. SUMIDA ; K. KATANO ; M. NAKAO.** *J. Oleo Sci.,* 2006, vol. 55, 653-656
- **D. A. FREY ; E. W. KATAISTO ; J. L. EKMANIS ; S. O'MALLEY ; S. F. LOCKWOOD.** *Org. Process Res. Dev.,* 2004, vol. 8, 796-801
- **H. SHANGGUAN ; S. ZHANG ; X. LI ; Q. ZHOU ; J. SHI ; Q. DENG ; F. HUANG.** *RSC Adv.,* 2020, vol. 10, 8949-8957
- **F. T. AHMAD ; D. E. MATHER ; H. Y. LAW ; M. LI ; S. A. J. YOUSIF ; K. J. CHALMERS ; R. E. ASENSTORFER ; D. J. MARES.** *J. Cereal Sci.,* 2015, vol. 64, 109-115
- **M. HOU ; R. WANG ; X. WU ; Y. ZHANG ; J. GE ; Z. LIU.** *Catal. Lett,* 2015, vol. 145, 1825-1829
- **Z. Q. LIU ; L. M. ZHOU ; P. LIU ; P. J. BAKER ; S. S. LIU ; Y. P. XUE ; M. XU ; Y. G. ZHENG.** *Appl. Microbiol. Biotechnol.,* 2015, vol. 99, 8891-8902
- **F. P. BYRNE ; S. JIN ; G. PAGGIOLA ; T. H. M. PETCHEY ; J. H. CLARK ; T. J. FARMER ; A. J. HUNT ; C. ROBERT MCELROY ; J. SHERWOOD.** *Sustain. Chem. Process,* 2016, vol. 4, 4-30
- **D. R. JOSHI ; N. ADHIKARI.** *J. Pharm. Res. Int.,* 2019, vol. 28, 1-18
- **D. PRAT, A. WELLS ; J. HAYLER ; H. SNEDDON ; C. R. MCELROY ; S. ABOU-SHEHADA ; P. J. DUNN.** *Green Chem,* 2016, vol. 18, 288-296
- **J. H. CLARK ; A. HUNT ; C. TOPI ; G. PAGGIOLA ; J. SHERWOOD.** Sustainable Solvents: Perspectives from Research, Business and International Policy: Green Chemistry Concepts and Metrics for Solvent Selection. Green Chemistry, Royal Society of Chemistry, 2017, 188-234
- **E. YARA-VARÓN ; A. SELKA ; A. S. FABIANO-TIXIER ; M. BALCELLS ; R. CANELA-GARAYOA ; A. BILY ; M. TOUAIBIA ; F. CHEMAT.** *Green Chem.,* 2016, vol. 18, 6596-6608
- **B. SALEHI ; S. UPADHYAY ; I. E. ORHAN ; A. K. JUGRAN ; S. L. D. JAYAWEERA ; D. A. DIAS ; F. SHAROPOV ; Y. TAHERI ; N. MARTINS ; N. BAGHALPOUR.** *Biomolecules,* 2019, vol. 9, 738
- **J. CAO ; L. CHEN ; M. LI ; F. CAO ; L. ZHAO ; E. SU.** *Green Chem.,* 2018, vol. 20, 1879-1886
- **T. LEMAOUI ; A. S. DARWISH ; A. ATTOUI ; F. A. HATAB ; N. E. H. HAMMOUDI ; Y. BENGUERBA ; L. F. VEGA ; I. M. ALNASHEF.** *Green Chem,* 2020, vol. 22, 8511-8530
- **M. W. NAM ; J. ZHAO ; M. S. LEE ; J. H. JEONG ; J. LEE.** *Green Chem,* 2015, vol. 17, 1718-1727
- **S. HONG ; Y. YUAN ; Q. YANG ; L. CHEN ; J. DENG ; W. CHEN ; H. LIAN ; J. D. MOTA-MORALES ; H. LIIMATAINEN.** *Carbohydr. Polym.,* 2019, vol. 220, 211-218
- *MarvinSketch (version 20.08),* 2018, http://www.chemaxon.com/products/marvin/marvinsketch
- **M. HÁDA ; V. NAGY ; J. DELI ; A. AGÓCS.** *Molecules,* 2012, vol. 17, 5003-5012
- **J. E. SANGER.** *Ecology,* 1971, vol. 52, 1075-1089
- **A. LINDEN ; B. BÜRGI ; C. H. EUGSTER.** *Helv. Chim. Acta,* 2004, vol. 87, 1254-1269
- **J. DELI ; E. OSZ.** *Arkivoc,* 2004, 150-168
- **D. E. BREITHAUPT ; A. BAMEDI.** *J. Agric. Food Chem.,* 2001, vol. 4, 2064-2070
- **G. NADOLSKI ; A. J. CARDOUNEL ; J. L. ZWEIER ; S. F. LOCKWOOD.** *Bioorganic Med. Chem. Lett,* 2006, vol. 16, 775-781
- **P. E. LUX ; R. CARLE ; L. ZACARÍAS ; M. J. RODRIGO ; R. M. SCHWEIGGERT ; C. B. STEINGASS.** *J. Agric. Food Chem.,* 2019, vol. 67, 13164-13175
- **F. C. PETRY ; A. Z. MERCADANTE.** *J. Agric. Food Chem.,* 2016, vol. 64, 8207-8224
- **C. F. AQUINO ; L. C. C. SALOMÃO ; H. M. PINHEIRO-SANT'ANA ; S. M. R. RIBEIRO ; D. L. DE SIQUEIRA ; P. R. CECON.** *Rev. Ceres,* 2018, vol. 65, 217-226
- **Q. Y. LU ; Y. ZHANG ; Y. WANG ; D. WANG ; R. P. LEE ; K. GAO ; R. BYRNS ; D. HEBER.** *J. Agric. Food Chem.,* 2009, vol. 57, 10408-10413
- **P. JIMENEZ ; P. GARCIA ; V. QUITRAL ; K. VASQUEZ ; C. PARRA-RUIZ ; M. REYES-FARIAS ; D. F. GARCIA-DIAZ ; P. ROBERT ; C. ENCINA ; J. SOTO-COVASICH.** *Food Rev. Int,* 2020
- **K. G. RANGANATH ; K. S. SHIVASHANKARA ; T. K. ROY ; M. R. DINESH ; G. A. GEETHA ; K. C. G. PAVITHRA ; K. V. RAVISHANKAR.** *J. Food Sci. Technol,* 2018, vol. 55, 4566-4577
- **C. MUSSAGY ; V. C. SANTOS-EBINUMA ; K. A. KURNIA ; A. DIAS ; P. J. CARVALHO ; J. A. P. COUTINHO ; J. F. B. PEREIRA.** *Green Chem.,* 2020, vol. 22, 8478-8494
- **S. MARZORATI ; A. SCHIEVANO ; A. IDÀ ; L. VEROTTA.** *Green Chem.,* 2020, vol. 22, 187-196